(19) Europäisches Patentamt
European Patent Office
Office européen des brevets

(11) Veröffentlichungsnummer: **0 566 020 A1**

(12) **EUROPÄISCHE PATENTANMELDUNG**

(21) Anmeldenummer: **93105770.7**

(22) Anmeldetag: **07.04.93**

(51) Int. Cl.[5]: **C07D 403/04**, C07D 413/04, C07D 413/14, C07D 403/14, C07D 235/08, A61K 31/415, A61K 31/42

(30) Priorität: **11.04.92 DE 4212250**
**27.07.92 DE 4224752**

(43) Veröffentlichungstag der Anmeldung:
**20.10.93 Patentblatt 93/42**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Ries, Uwe, Dr., Dipl.-Chem.**
**Dunantstrasse 10**
**W-7950 Biberach 1(DE)**

Erfinder: **Hauel, Norbert, Dr., Dipl.-Chem.**
**Marderweg 12**
**W-7957 Schemmerhofen(DE)**
Erfinder: **Narr, Berthold, Dr., Dipl.-Chem.**
**Obere Au 5**
**W-7950 Biberach 1(DE)**
Erfinder: **van Meel, Jacques, Dr.**
**Schubertweg 4**
**W-7951 Mittelbiberach(DE)**
Erfinder: **Wienen, Wolfgang, Dr., Dipl.-Biologe**
**Am Schiessberg 28**
**W-7951 Äpfingen(DE)**
Erfinder: **Entzeroth, Michael, Dr., Dipl.-Chem.**
**Sebastian-Sailer-Strasse 44**
**W-7951 Warthausen(DE)**

(54) **Benzimidazole, diese Verbindungen enthaltende Arzneimittel und Verfahren zu ihrer Herstellung.**

(57) Die Erfindung betrifft substituierte Benzimidazole der allgemeinen Formel

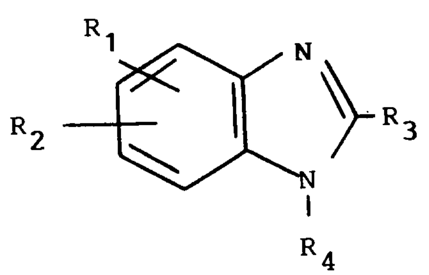

,(I)

in der
$R_1$ bis $R_4$ wie im Anspruch 1 definiert sind, deren 1-, 3-Isomerengemische, deren quartäre N-Alkylsalze und deren Salze, welche wertvolle Eigenschaften aufweisen.

Diese weisen wertvolle pharmakologische Eigenschaften auf, insbesondere Angiotensin-antagonistische Wirkungen, vorzugsweise Angiotensin-II-antagonistische Wirkungen.

EP 0 566 020 A1

In der EP-A-0 468 470 werden bereits Benzimidazole, welche wertvolle Angiotensin-Antagonisten darstellen, beschrieben.

Es wurde nun gefunden, daß die neuen Benzimidazole der allgemeinen Formel

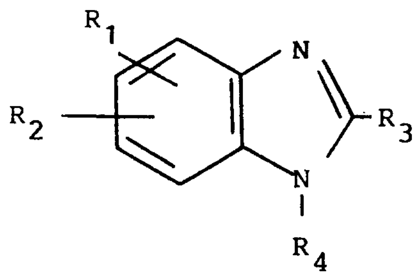

, (I)

deren 3-Isomere, deren 1-, 3-Isomerengemische und deren Salze wertvolle Eigenschaften aufweisen.

In der obigen allgemeinen Formel I bedeutet

$R_1$ ein Wasserstoff-, Fluor-, Chlor- oder Bromatom, eine Fluormethyl-, Difluormethyl-, Trifluormethyl- oder Alkylgruppe,

$R_2$ eine gegebenenfalls in 1-Stellung durch den Rest $R_a$ substituierte Imidazol-2-yl-Gruppe, wobei

$R_a$ eine Phenyl- oder Phenylalkylgruppe, in der der Phenylkern jeweils durch Alkyl-, Hydroxy- oder Alkoxygruppen mono- oder disubstituiert sein kann und die Substituenten gleich oder verschieden sein können, eine Cycloalkylgruppe mit 3 bis 7 Kohlenstoffatomen oder eine Alkylgruppe mit 1 bis 6 Kohlenstoffatomen, in der der Alkylteil zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe oder ab Position 2 durch eine Hydroxy-, Alkoxy-, Amino-, Alkylamino-, Dialkylamino-, Pyrrolidino-, Piperidino- oder Morpholinogruppe substituiert sein kann, darstellt,

eine 5,5-Spiro-cyclopentano-dihydro-imidazol-4-on-2-yl-gruppe,

eine in 1- und 3-Stellung durch den Rest $R_b$, der gleich oder verschieden sein kann, substituierte Imidazolium-2-yl-Gruppe, wobei

$R_b$ eine Phenylalkylgruppe, in der der Phenylkern durch Alkyl-, Hydroxy- oder Alkoxygruppen mono- oder disubstituiert sein kann und die Substituenten gleich oder verschieden sein können, oder eine Alkylgruppe mit 1 bis 6 Kohlenstoffatomen, in der der Alkylteil zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, darstellt,

eine Oxazol-2-yl- oder Thiazol-2-yl-Gruppe, wobei in den vorstehend erwähnten Imidazol-2-yl-, Imidazolium-2-yl-, Oxazol-2-yl- oder Thiazol-2-yl-teilen jeweils die 4-, 5-Positionen durch eine Alkylgruppe mit 1 bis 5 Kohlenstoffatomen oder durch eine Phenylgruppe substituiert sein können, wobei die Substituenten gleich oder verschieden sein können, oder auch über die 4,5-Positionen eine n-Propylen- oder n-Butylenbrücke angefügt sein kann,

eine in 4-Stellung durch $R_9$ und $R_{10}$ und in 5-Stellung durch $R_{10}$ substituierte Oxazolin-2-yl- oder Imidazolin-2-yl-Gruppe,wobei eine Iminogruppe zusätzlich durch $R_a$ substituiert sein kann, oder eine $R_8CO-(R_9CR_{10})-NR_a-CO$-Gruppe, in denen

$R_a$ wie vorstehend erwähnt definiert ist,

$R_8$ bis $R_{10}$, die gleich oder verschieden sein können, Wasserstoffatome, Alkylgruppen mit 1 bis 5 Kohlenstoffatomen oder Phenylgruppen darstellen,

$R_3$ eine Alkylgruppe mit 1 bis 5 Kohlenstoffatomen, eine Cycloalkylgruppe mit 3 bis 5 Kohlenstoffatomen, eine Alkoxy- oder Alkylthiogruppe mit jeweils 1 bis 4 Kohlenstoffatomen und

$R_4$ ein Wasserstoffatom oder eine Gruppe der allgemeinen Formel

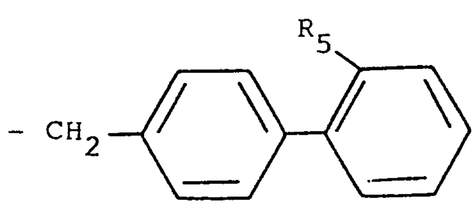

,

in welcher

$R_5$ eine in vivo in eine Carboxygruppe metabolisierbare Gruppe, eine Carboxy-, Cyano-, 2,5-Dihydro-5-oxo-1,2,4-oxadiazol-3-yl-, 1H-Tetrazolyl-, 1-Triphenylmethyl-tetrazolyl- oder 2-Triphenylmethyl-tetrazolylgruppe darstellt,

wobei alle vorstehend erwähnten Alkyl- und Alkoxygruppen, soweit nichts anderes erwähnt wurde, jeweils 1 bis 3 Kohlenstoffatome enthalten können und

unter dem vorstehend erwähnten Begriff "eine in-vivo in eine Carboxygruppe metabolisierbare Gruppe" beispielsweise deren Ester der Formeln

- CO - OR',
- CO - O - (HCR'') - O - CO - R''' und
- CO - O - (HCR'') - O - CO - OR'''

in denen

R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,

R'' ein Wasserstoffatom oder eine Methylgruppe und

R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen sind.

Die neuen Verbindungen der allgemeinen Formel I, in denen $R_5$ eine Gruppe, die in-vivo metabolisch in eine Carboxygruppe umgewandelt wird, eine Carboxy- oder 1H-Tetrazolylgruppe bedeutet, weisen insbesondere wertvolle pharmakologische Eigenschaften auf, da diese Angiotensin-Antagonisten, insbesondere Angiotensin-II-Antagonisten, darstellen. Die übrigen Verbindungen der allgemeinen Formel I, in denen $R_4$ ein Wasserstoffatom oder $R_5$ die Cyano-, 1-Triphenylmethyltetrazolyl- oder 2-Triphenylmethyl-tetrazolylgruppe darstellen, stellen wertvolle Zwischenprodukte zur Herstellung der vorstehend erwähnten Verbindungen dar.

Gegenstand der vorliegenden Erfindung sind somit die neuen Zwischenprodukte der obigen allgemeinen Formel I und deren Salze, die neuen Angiotensin-II-Antagonisten der obigen allgemeinen Formel I und deren physiologisch verträglichen Salze, diese Verbindungen enthaltende Arzneimittel, welche aufgrund der oben erwähnten pharmakologischen Eigenschaften zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen, Blasenerkrankungen, zur Prävention atherosklerotischer Gefäßveränderungen und zur Prävention von Restenosen nach operativer Erweiterung von Blutgefäß-Stenosen geeignet sind, und Verfahren zu ihrer Herstellung.

Für die bei der Definition der Reste $R_1$ bis $R_5$ eingangs erwähnten Bedeutungen kommt beispielsweise für $R_1$ die Bedeutung des Wasserstoff-, Fluor-, Chlor- oder Bromatoms, der Fluormethyl-, Difluormethyl-, Trifluormethyl-, Methyl-, Ethyl-, n-Propyl- oder Isopropylgruppe,

für $R_2$ die der 5,5-Spiro-cyclopentano-dihydro-imidazol-4-on-2-yl-, 1-Methyl-5,5-spiro-cyclopentano-dihydro-imidazol-4-on-2-yl-, 1-Ethyl-5,5-spiro-cyclopentano-dihydro-imidazol-4-on-2-yl-, 1-n-Propyl-5,5-spiro-cyclopentano-dihydro-imidazol-4-on-2-yl-,1-Isopropyl-5,5-spiro-cyclopentano-dihydro-imidazol-4-on-2-yl-,4,5-Dimethyl-oxazol-2-yl-, 4-Methyl-5-ethyl-oxazol-2-yl-, 4-Methyl-5-n-propyl-oxazol-2-yl-, 4-Methyl-5-n-butyl-oxazol-2-yl-, 4-Methyl-5-n-pentyl-oxazol-2-yl-, 4-Methyl-5-phenyl-oxazol-2-yl-, 4,5-Diethyl-oxazol-2-yl-, 4-Ethyl-5-n-propyl-oxazol-2-yl-, 4-Ethyl-5-n-butyl-oxazol-2-yl-, 4-Ethyl-5-n-pentyl-oxazol-2-yl-, 4-Ethyl-5-phenyl-oxazol-2-yl-, 4,5-Di-n-propyl-oxazol-2-yl-, 4-n-Propyl-5-n-butyl-oxazol-2-yl-, 4-n-Propyl-5-n-pentyl-oxazol-2-yl-, 4-n-Propyl-5-phenyl-oxazol-2-yl-, 5-Methyl-4-ethyl-oxazol-2-yl-, 5-Methyl-4-n-propyl-oxazol-2-yl-, 4,5-Diphenyl-oxazol-2-yl-,5,6,7,8-Tetrahydro-benzoxazol-2-yl-, 4,5-Dimethyl-thiazol-2-yl-, 4-Methyl-5-ethyl-thiazol-2-yl-, 4-Methyl-5-n-propyl-thiazol-2-yl-, 4-Methyl-5-phenyl-thiazol-2-yl-, 4,5-Diethyl-thiazol-2-yl-, 4-Ethyl-5-n-propyl-thiazol-2-yl-, 4-Ethyl-5-phenyl-thiazol-2-yl-, 4,5-Di-n-propyl-thiazol-2-yl-, 4,5-Di-n-isopropyl-thiazol-2-yl-, 4-n-Propyl-5-phenyl-thiazol-2-yl-, 4,5-Diphenyl-thiazol-2-yl-, 5,6,7,8-Tetrahydro-benz-thiazol-2-yl-,4-Methyl-oxazolin-2-yl-, 4-Ethyl-oxazolin-2-yl-, 4-n-Propyl-oxazolin-2-yl-, 4-Isopropyl-oxazolin-2-yl-, 4-n-Butyl-oxazolin-2-yl-, 4-Isobutyl-oxazolin-2-yl-, 4-Benzyl-oxazolin-2-yl-, 4-Phenyl-oxazolin-2-yl-, 4,4-Dimethyl-oxazolin-2-yl-, 4-Methyl-5-phenyl-oxazolin-2-yl-, 4,4-Dimethyl-5-n-propyl-oxazolin-2-yl-, 4,5-Tetramethylen-oxazolin-2-yl-, 4-Methyl-imidazolin-2-yl-, 4,5-Dimethyl-imidazolin-2-yl-, 4,5-Tetramethylen-imidazolin-2-yl-, 4-Methyl-imidazol-2-yl-, 4,5-Dimethyl-imidazol-2-yl-, 1,4,5-Trimethyl-imidazol-2-yl-, 1-Ethyl-4,5-dimethyl-imidazol-2-yl-, 1-n-Propyl-4,5-dimethyl-imidazol-2-yl-, 1-Isopropyl-4,5-dimethyl-imidazol-2-yl-, 1-n-Butyl-4,5-dimethyl-imidazol-2-yl-, 1-Isobutyl-4,5-dimethyl-imidazol-2-yl-, 1-Cyclopropyl-4,5-dimethyl-imidazol-2-yl-, 1-Cyclobutyl-4,5-dimethyl-imidazol-2-yl-, 1-Cyclopentyl-4,5-dimethyl-imidazol-2-yl-, 1-Cyclohexyl-4,5-dimethyl-imidazol-2-yl-, 1-Cycloheptyl-4,5-dimethyl-imidazol-2-yl-, 1-Benzyl-4,5-dimethyl-imidazol-2-yl-, 1-(2-Phenyl-ethyl)-4,5-dimethyl-imidazol-2-yl-, 1-Carboxymethyl-4,5-dimethyl-imidazol-2-yl-, 3-Methoxycarbonylmethyl-4,5-dimethyl-imidazol-2-yl-, 1-Ethoxycarbonylmethyl-4,5-dimethyl-imidazol-2-yl-, 1-n-Propoxycarbonylmethyl-4,5-dimethyl-imidazol-2-yl-, 1-Isopropoxycarbonylmethyl-4,5-dimethyl-imidazol-2-yl-, 1-Aminocarbonylmethyl-4,5-dimethyl-imidazol-2-yl-, 1-Methylaminocarbonylmethyl-4,5-dimethyl-imidazol-2-yl-, 1-Ethylaminocarbonylmethyl-4,5-dimethyl-imidazol-2-yl-, 1-n-Propylaminocarbonylmethyl-4,5-dimethyl-imidazol-2-yl-, 1-Isopropylaminocarbonylmethyl-4,5-dimethyl-imidazol-2-yl-, 1-Dimethylaminocarbonylmethyl-4,5-dimethyl-imidazol-2-yl-, 1-Diethylaminocarbonylmethyl-4,5-dimethyl-imidazol-2-yl-, 1-Di-n-propylaminocarbonylmethyl-4,5-dimethylimidazol-2-yl-, 1-Diisopropylaminocarbonylmethyl-4,5-dimethyl-imidazol-2-yl-, 1-N-Methyl-ethylaminocarbonylmethyl-4,5-dimethyl-imidazol-2-yl-, 1-(2-Carboxy-ethyl)-4,5-dimethyl-imidazol-2-yl-, 1-(2-Methoxycarbonyl-ethyl)-4,5-dimethyl-imidazol-2-yl-, 1-(2-Ethoxycarbonyl-ethyl)-4,5-dimethyl-imidazol-2-yl-, 1-(2-n-Propoxycarbonyl-ethyl)-4,5-dimethyl-imidazol-2-yl-, 1-(2-Isopropoxycarbonyl-ethyl)-4,5-dimethyl-imidazol-2-yl-, 1-(2-Aminocarbonyl-ethyl)-4,5-dimethylimidazol-2-yl-, 1-(2-Methylaminocarbonyl-ethyl)-4,5-dimethyl-imidazol-2-yl-, 1-(2-Ethylaminocarbonyl-ethyl)-4,5-dimethyl-imidazol-2-yl-, 1-(2-n-Propylaminocarbonyl-ethyl)- 4,5-dimethyl-imidazol-2-yl-, 1-(2-Isopropylaminocarbonylethyl)-4,5-dimethyl-imidazol-2-yl-, 1-(2-Dimethylaminocarbonyl-ethyl)-4,5-dimethyl-imidazol-2-yl-, 1-(2-Diethylaminocarbonyl-ethyl)-4,5-dimethyl-imidazol-2-yl-, 1-(2-Di-n-propylaminocarbonyl-ethyl)-4,5-dimethyl-imidazol-2-yl-,1-(2-Diisopropylaminocarbonyl-ethyl)-4,5-dimethyl-imidazol-2-yl-, 1-(2-N-Methyl-ethylamino-carbonyl-ethyl)-4,5-dimethyl-imidazol-2-yl-, 1-(3-Carboxy-propyl)-4,5-dimethyl-imidazol-2-yl-, 1-(3-Methoxycarbonyl-propyl)-4,5-dimethyl-imidazol-2-yl-, 1-(3-Ethoxycarbonyl-propyl)-4,5-dimethyl-imidazol-2-yl-, 1-(3-n-Propoxycarbonyl-propyl)-4,5-dimethyl-imidazol-2-yl-, 1-(3-Isopropoxycarbonyl-propyl)-4,5-dimethyl-imidazol-2-yl-, 1-(3-Aminocarbonyl-propyl)-4,5-dimethyl-imidazol-2-yl-, 1-(3-Methylaminocarbonyl-propyl)-4,5-dimethyl-imidazol-2-yl-, 1-(3-Ethylaminocarbonyl-propyl)-4,5-dimethyl-imidazol-2-yl-, 1-(3-n-Propylaminocarbonyl-propyl)-4,5-dimethyl-imidazol-2-yl-, 1-(3-Isopropylaminocarbonylpropyl)-4,5-dimethyl-imidazol-2-yl-, 1-(3-Dimethylaminocarbonyl-propyl)-4,5-dimethyl-imidazol-2-yl-, 1-(3-Diethylaminocarbonyl-propyl)-4,5-dimethyl-imidazol-2-yl-, 1-(3-Di-n-propylaminocarbonyl-propyl)-4,5-dimethyl-imidazol-2-yl-,1-(3-Diisopropylaminocarbonyl-propyl)-4,5-dimethyl-imidazol-2-yl-, 1-(3-N-Methyl-ethylaminocarbonyl-propyl)-4,5-dimethyl-imidazol-2-yl-, 1-(2,2,2-Trifluorethyl)-4,5-dimethyl-imidazol-2-yl-, 1-(2-Hydroxyethyl)-4,5-dimethyl-imidazol-2-yl-, 1-(3-Hydroxypropyl)-4,5-dimethyl-imidazol-2-yl-, 1-(4-Hydroxybutyl)-4,5-dimethyl-imidazol-2-yl-, 1-(2-Methoxyethyl)-4,5-dimethyl-imidazol-2-yl-, 1-(3-Methoxypropyl)-4,5-dimethyl-imidazol-2-yl-, 1-(4-Methoxybutyl)-4,5-dimethyl-imidazol-2-yl-, 1-(2-Ethoxyethyl)-4,5-dimethyl-imidazol-2-yl-, 1-(3-Ethoxypropyl)-4,5-dimethyl-imidazol-2-yl-, 1-(4-Ethoxybutyl)-4,5-dimethyl-imidazol-2-yl-, 1-(2-Isopropoxyethyl)-4,5-dimethyl-imidazol-2-yl-, 1-(3-n-Propoxypropyl)-4,5-dimethyl-imidazol-2-yl-, 1-(4-Isopropoxybutyl)-4,5-dimethyl-imidazol-2-yl-, 1-(2-Pyrrolidinoethyl)-4,5-dimethyl-imidazol-2-yl-, 1-(3-Pyrrolidinopropyl)-4,5-dimethyl-imidazol-2-yl-, 1-(4-Pyrrolidinobutyl)-4,5-dimethyl-imidazol-2-yl-, 1-(2-Piperidinoethyl)-4,5-dimethyl-imidazol-2-yl-, 1-(3-Piperidinopropyl)-4,5-dimethyl-imidazol-2-yl-, 1-(4-Piperidinobutyl)-4,5-dimethyl-imidazol-2-yl-, 1-(2-Morpholinoethyl)-4,5-dimethyl-imidazol-2-yl-, 1-(3-Morpholinopropyl)-4,5-dimethyl-imidazol-2-yl-, 1-(4-Morpholinobutyl)-4,5-dimethyl-imidazol-2-yl-, 1-Phenyl-4,5-dimethyl-imidazol-2-yl-, 1-Benzyl-4,5-dimethyl-imidazol-2-yl-, 1-(1-Phenylethyl)-4,5-dimethyl-imidazol-2-yl-, 1-(2-Phenylethyl)-4,5-dimethyl-imidazol-2-yl-, 1-(1-Phenylpropyl)-4,5-dimethyl-imidazol-2-yl-, 1-(2-Phenylpropyl)-4,5-dimethyl-imidazol-2-yl-, 1-(3-Phenylpropyl)-4,5-dimethyl-imidazol-2-yl-, 1-Methyl-4,5-diethyl-imidazol-2-yl-, 1,4,5-Triethyl-imidazol-2-yl-, 1-Ethyl-4-isopropyl-5-methyl-imidazol-2-yl-, 1-Ethyl-4-isobutyl-5-methyl-imidazol-2-yl-, 1-n-Propyl-4-isopropyl-5-methyl-imidazol-2-yl-, 1-n-Propyl-4-isobutyl-5-methyl-imidazol-2-yl-, 1,4-Diisopropyl-5-methyl-imidazol-2-yl-, 1-Isopropyl-4-isobutyl-5-methyl-imidazol-2-yl-, 1-(2-Dimethylamino-ethyl)-4-isopropyl-5-methyl-imidazol-2-yl-, 1-(2-Dimethylamino-ethyl)-4-isobutyl-5-methyl-imidazol-2-yl-, 1-(3-Dimethylamino-propyl)-4-isopropyl-5-methyl-imidazol-2-yl-,1-(3-Dimethylamino-propyl)-4-isobutyl-5-methyl-imidazol-2-yl-, 1,5-Dimethyl-4-ethyl-imidazol-2-yl-, 1,5-Dimethyl-4-n-propyl-imidazol-2-yl-, 1,5-Dimethyl-4-isopropyl-imidazol-2-yl-, 1,5-Dimethyl-4-isobutyl-imidazol-2-yl-, 1,5-Dimethyl-4-phenyl-imidazol-2-yl-, 1-Methyl-4,5-diphenyl-imidazol-2-yl-, 1-Ethyl-4,5-diphenyl-imidazol-2-yl-, 1-n-Propyl-4,5-diphenyl-imidazol-2-yl-, 1-Isopropyl-4,5-diphenyl-imidazol-2-yl-, 1-Carboxymethyl-4,5-diphenyl-imidazol-2-yl-, 1-Methoxycarbonyl-methyl-4,5-diphenyl-imidazol-2-yl-, 1-Ethoxycarbonylmethyl-4,5-diphenyl-imidazol-2-yl-, 4,5-Trimethylen-imidazol-2-yl-, 1-Methyl-4,5-trimethylen-imidazol-2-yl-, 5,6,7,8-Tetrahydro-benzimidazol-2-yl-, 1-Methyl-5,6,7,8-tetrahydro-benzimidazol-2-yl-, 1-Ethyl-5,6,7,8-tetrahydro-benzimidazol-2-yl-, 1-n-Propyl-5,6,7,8-tetrahydro-benzimidazol-2-yl-, 1-Isopropyl-5,6,7,8-tetrahydro-benzimidazol-2-yl-, 1-n-Butyl-5,6,7,8-tetrahydro-benzimidazol-2-yl-, 1-Isobutyl-5,6,7,8-tetrahydro-benzimidazol-2-yl-, 1-n-Pentyl-5,6,7,8-tetrahydro-benzimidazol-2-yl-, 1-n-Hexyl-5,6,7,8-tetrahydro-benzimidazol-2-yl-, 1-Cyclopropyl-5,6,7,8-tetrahydro-benzimidazol-2-yl-, 1-Cyclobutyl-5,6,7,8-tetrahydro-benzimidazol-2-yl-, 1-Cyclopentyl-5,6,7,8-tetrahydro-benzimidazol-2-yl-, 1-Cyclohexyl-5,6,7,8-tetrahydro-benzimidazol-2-yl-, 1-Cycloheptyl-5,6,7,8-tetrahydro-benzimidazol-2-yl-, 1-Carboxymethyl-5,6,7,8-tetrahydro-benzimidazol-2-yl-, 1-Methoxycarbonylmethyl-5,6,7,8-tetrahydro-benzimidazol-2-yl-,1-Ethoxycarbonylmethyl-5,6,7,8-tetrahydro-benzimidazol-2-yl-, 1-n-propoxycarbonylmethyl-5,6,7,8-tetrahydro-benzimidazol-2-yl-, 1-Isopropoxycarbonylmethyl-5,6,7,8-tetrahydro-benzimidazol-2-yl-, 1-Aminocarbonylmethyl-5,6,7,8-tetrahydro-benzimidazol-2-yl-, 1-Methylaminocarbonylmethyl-5,6,7,8-tetrahydro-benzimidazol-2-yl-, 1-Ethylaminocarbonylmethyl-5,6,7,8-tetrahydro-benzimidazol-2-yl-, 1-n-Propylaminocarbonylmethyl-5,6,7,8-tetrahydro-benzimidazol-2-yl-, 1-Isopropylaminocarbonylmethyl-5,6,7,8-tetrahydro-benzimidazol-2-yl-, 1-Dimethylaminocarbonylmethyl-5,6,7,8-tetrahydro-benzimidazol-2-yl-,1-Diethylaminocarbonylmethyl-5,6,7,8-tetrahydro-benzimidazol-2-yl-, 1-Di-n-propylaminocarbonylmethyl-5,6,7,8-tetrahydro-benzimidazol-2-yl-, 1-Diisopropylaminocarbonylmethyl-5,6,7,8-tetrahydro-benzimidazol-2-yl-, 1-N-Methyl-ethylaminocarbonyl-methyl-5,6,7,8-tetrahydro-benzimidazol-2-yl-, 1-(2-Carboxy-ethyl)-5,6,7,8-tetrahydro-benzimidazol-2-yl-, 1-(2-Methoxycarbonyl-ethyl)-5,6,7,8-tetrahydro-benzimidazol-2-yl-, 1-(2-Ethoxycarbonyl-ethyl)-5,6,7,8-tetrahydro-benzimidazol-2-yl-, 1-(2-n-Propoxycarbonyl-ethyl)-5,6,7,8-tetrahydro-benzimidazol-2-yl-,1-(2-Isopropoxycarbonyl-ethyl)-5,6,7,8-tetrahydro-benzimidazol-2-yl-,1-(2-Aminocarbonyl-ethyl)-5,6,7,8-tetrahydro-benzimidazol-2-yl-, 1-(2-Methylaminocarbonyl-ethyl)-5,6,7,8-tetrahydro-benzimidazol-2-yl-,1-(2-Ethylami-nocarbonyl-ethyl)-5,6,7,8-tetrahydro-benzimidazol-2-yl-, 1-(2-n-Propylaminocarbonylethyl)-5,6,7,8-tetrahydro-benzimidazol-2-yl-, 1-(2-Isopropylaminocarbonyl-ethyl)-5,6,7,8-tetrahydro-benzimidazol-2-yl-, 1-(2-Dimethylaminocarbonyl-ethyl)-5,6,7,8-tetrahydro-benzimidazol-2-yl-, 1-(2-Diethylaminocarbonyl-ethyl)-5,6,7,8-tetrahydro-benzimidazol-2-yl-, 1-(2-Di-n-propylaminocarbonylethyl)-5,6,7,8-tetrahydro-benzimidazol-2-yl-, 1-(2-Diisopropylaminocarbonyl-ethyl)-5,6,7,8-tetrahydro-benzimidazol-2-yl-, 1-(2-N-Methyl-ethylaminocarbonyl-ethyl)-5,6,7,8-tetrahydro-benzimidazol-2-yl-, 1-(3-Carboxy-propyl)-5,6,7,8-tetrahydro-benzimidazol-2-yl-, 1-(3-Methoxycarbonyl-propyl)-5,6,7,8-tetrahydro-benzimidazol-2-yl-,1-(3-Ethoxycarbonylpropyl)-5,6,7,8-tetrahydro-benzimidazol-2-yl-, 1-(3-n-Propoxycarbonyl-propyl)-5,6,7,8-tetrahydro-benzimidazol-2-yl-, 1-(3-Isopropoxycarbonyl-propyl)-5,6,7,8-tetrahydro-benzimidazol-2-yl-,1-(3-Aminocarbonyl-propyl)-5,6,7,8-tetrahydrobenzimidazol-2-yl-, 1-(3-Methylaminocarbonyl-propyl)-5,6,7,8-tetrahydro-benzimidazol-2-yl-, 1-(3-Ethylaminocarbonyl-propyl)-5,6,7,8-tetrahydro-benzimidazol-2-yl-,1-(3-n-Propylaminocarbonyl-propyl)-5,6,7,8-tetrahydro-benzimidazol-2-yl-, 1-(3-Isopropylaminocarbonyl-propyl)-5,6,7,8-tetrahydro-benzimidazol-2-yl-, 1-(3-Dimethylaminocarbonyl-propyl)-5,6,7,8-tetrahydro-benzimidazol-2-yl-, 1-(3-Diethylaminocarbonyl-propyl)-5,6,7,8-tetrahydro-benzimid-azol-2-yl-, 1-(3-Di-n-propylaminocarbonyl-propyl)-5,6,7,8-tetrahydro-benzimidazol-2-yl-, 1-(3-Diisopropylaminocarbonyl-propyl)-5,6,7,8-tetrahydro-benzimidazol-2-yl-, 1-(3-N-Methyl-ethylaminocarbonyl-propyl)-5,6,7,8-tetrahydro-benzimidazol-2-yl-, 1-(2,2,2-Trifluorethyl)-5,6,7,8-tetrahydro-benzimidazol-2-yl-, 1-Phenyl-5,6,7,8-tetrahydro-benzimidazol-2-yl-, 1-Benzyl-5,6,7,8-tetrahydro-benzimidazol-2-yl-, 1-(1-Phenylethyl)-5,6,7,8-tetrahydro-benzimidazol-2-yl-, 1-(2-Phenylethyl)-5,6,7,8-tetrahydro-benzimidazol-2-yl-, 1-(1-Phenylpropyl)-5,6,7,8-tetrahydro-benzimidazol-2-yl-, 1-(2-Phenylpropyl)-5,6,7,8-tetrahydro-benzimidazol-2-yl-, 1-(3-Phenylpropyl)-5,6,7,8-tetrahydro-benzimidazol-2-yl-, 1,3-Dimethyl-5,6,7,8-tetrahydro-benzimidazolium-2-yl-, 1,3-Diethyl-5,6,7,8-tetrahydro-benzimidazolium-2-yl-, 1,3-Di-n-propyl-5,6,7,8-tetrahydro-benzimidazolium-2-yl- oder 1,3-Dibenzyl-5,6,7,8-tetrahydro-benzimidazolium-2-yl-Gruppe,

für $R_3$ die der Methyl-, Ethyl-, n-Propyl-, Isopropyl-, Isobutyl-, n-Butyl-, 1-Methyl-n-propyl-, tert.Butyl-, n-Pentyl-, 1-Methyl-n-butyl-, 2-Methyl-n-butyl-, Cyclopropyl-, Cyclobutyl-, Cyclopentyl-, Methoxy-, Ethoxy-, n-Propoxy-, Isopropoxy-, n-Butoxy-, Isobutoxy-, Methylthio-, Ethylthio-, n-Propylthio-, Isopropylthio-, n-Butylthio- oder Isobutylthiogruppe und

für $R_5$ die der Carboxy-, Cyano-, 1H-Tetrazolyl-, 1-Triphenylmethyl-tetrazolyl-, 2-Triphenylmethyl-tetrazolyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 1-Phenylpropionyloxymethoxycarbonyl-, 2-Phenylpropionyloxymethoxycarbonyl-, 3-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-,n-Butyloxycarbonyloxymethoxycarbonyl-, Isobutyloxycarbonyloxymethoxycarbonyl-, tert.Butyloxycarbonyloxymethoxycarbonyl-,n-Pentyloxycarbonyloxymethoxycarbonyl-, Isoamyloxycarbonyloxymethoxycarbonyl-, n-Hexyloxycarbonyloxymethoxycarbonyl-, Cyclopentyloxycarbonyloxymethoxycarbonyl-, Cyclohexyloxycarbonyloxymethoxycarbonyl-, Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Aminocarbonyl-, Methylaminocarbonyl-, Ethylaminocarbonyl-, n-Propylaminocarbonyl-, Isopropylaminocarbonyl-, Dimethylaminocarbonyl-, Diethylaminocarbonyl-, Di-n-propylaminocarbonyl-, Diisopropylaminocarbonyl-, N-Methyl-ethylaminocarbonyl-, N-Ethyl-isopropylaminocarbonyl- oder 2,5-Dihydro-5-oxo-1,2,4-oxadiazol-3-yl-Gruppe in Betracht.

Bevorzugte Verbindungen der obigen allgemeinen Formel I sind diejenigen, in denen

$R_1$ ein Wasserstoff-, Fluor-, Chlor- oder Bromatom, eine Trifluormethylgruppe oder eine Alkylgruppe mit 1 bis 3 Kohlenstoffatomen,

$R_2$ eine gegebenenfalls in 1-Stellung durch den Rest $R_a$ substituierte Imidazol-2-yl-Gruppe, wobei

$R_a$ eine Phenylgruppe, eine Phenylalkylgruppe mit 1 bis 3 Kohlenstoffatomen im Alkylteil, eine Cycloalkylgruppe mit 5 bis 7 Kohlenstoffatomen oder eine Alkylgruppe mit 1 bis 5 Kohlenstoffatomen, in der der Alkylteil zusätzlich durch eine in vivo in eine Carboxygruppe metabolisierbare Gruppe, durch eine Trifluormethyl-, Carboxyl-, Methoxycarbonyl-, Ethoxycarbonyl-, Aminocarbonyl-, Methylaminocarbonyl- oder Dimethylaminocarbonylgruppe oder ab Position 2 durch eine Hydroxy-, Methoxy-, Ethoxy-, Amino-, Methylamino-, Dimethylamino-, Pyrrolidino-, Piperidino- oder Morpholinogruppe substituiert sein kann, darstellt,

eine 5,5-Spiro-cyclopentano-dihydro-imidazol-4-on-2-yl-gruppe,

eine in 1- und 3-Stellung durch den Rest $R_b$, der gleich oder verschieden sein kann, substituierte Imidazolium-2-yl-Gruppe, wobei

$R_b$ eine Alkyl- oder Phenylalkylgruppe mit jeweils 1 bis 3 Kohlenstoffatomen im Alkylteil darstellt,

eine Oxazol-2-yl- oder Thiazol-2-yl-Gruppe, wobei in den vorstehend erwähnten Imidazol-2-yl-, Imidazolium-2-yl-, Oxazol2-yl- oder Thiazol-2-yl-teilen jeweils die 4-, 5-Positionen durch eine Alkylgruppe mit 1 bis 4 Kohlenstoffatomen oder durch eine Phenylgruppe substituiert sein können, wobei die Substituenten gleich oder verschieden sein können, oder auch über die 4,5-Positionen eine n-Butylenbrücke angefügt sein kann, eine durch $R_9$ bis $R_{10}$ substituierte Oxazolin-2-yl- oder Imidazolin-2-yl-Gruppe, wobei zusätzlich eine Iminogruppe durch $R_a$ substituiert sein kann und $R_9$ bis $R_{10}$ und $R_a$ jeweils ein Wasserstoffatom oder eine Alkylgruppe mit 1 bis 4 Kohlenstoffatomen darstellen,

$R_3$ eine Alkylgruppe mit 2 bis 5 Kohlenstoffatomen, eine Cycloalkylgruppe mit 3 bis 5 Kohlenstoffatomen, eine Alkoxy- oder Alkylthiogruppe mit jeweils 2 bis 4 Kohlenstoffatomen und

$R_4$ eine Gruppe der allgemeinen Formel

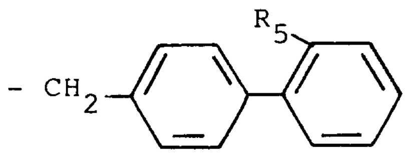

,

in welcher

$R_5$ eine in vivo in eine Carboxygruppe metabolisierbare Gruppe, eine Carboxy-, Cyano-, 2,5-Dihydro-5-oxo-1,2,4-oxadiazol-3-yl-, 1H-Tetrazolyl-, 1-Triphenylmethyl-tetrazolyl- oder 2-Triphenylmethyl-tetrazolylgruppe darstellt,
wobei unter dem vorstehend erwähnten Begriff "eine in-vivo in eine Carboxygruppe metabolisierbare Gruppe" beispielsweise deren Ester der Formeln

- CO - OR',
- CO - O - (HCR'') - O - CO - R''' und
- CO - O - (HCR'') - O - CO - OR'''

in denen
R' eine geradkettige oder verzweigte Alkylgruppe mit 1 bis 4 Kohlenstoffatomen, eine Cycloalkylgruppe mit 5 bis 7 Kohlenstoffatomen,
R'' ein Wasserstoffatom oder eine Methylgruppe und
R''' eine geradkettige oder verzweigte Alkylgruppe mit 1 bis 4 Kohlenstoffatomen oder eine Cycloalkylgruppe mit 5 bis 7 Kohlenstoffatomen bedeuten, zu verstehen sind,
deren 3-Isomere, 1-, 3-Isomerengemische und deren Salze.

Besonders bevorzugte Verbindungen der obigen allgemeinen Formel I, sind
diejenigen, in denen $R_3$ und $R_4$ wie vorstehend erwähnt definiert sind,
$R_2$ in 6-Stellung die vorstehend erwähnten Bedeutungen aufweist und
$R_1$ in 4-Stellung ein Fluor-, Chlor- oder Bromatom, eine Trifluormethylgruppe oder eine Alkylgruppe mit 1 bis 3 Kohlenstoffatomen bedeuten,
insbesondere diejenigen Verbindungen der obigen allgemeinen Formel I, in der $R_2$ in 6-Stellung einen der vorstehend erwähnten Imidazolylreste darstellt,
deren 3-Isomere, 1-, 3-Isomerengemische und deren Salze.

Als besonders bevorzugte Verbindungen seien folgende erwähnt:

(a) 4'-[[2-n-Propyl-4-methyl-6-(5,6,7,8-tetrahydrobenzimidazol-2-yl)-1H-benzimidazol-1-yl]-methyl]-biphenyl-2-carbonsäure,
(b) 4'-[[2-n-Propyl-4-methyl-6-(1-methyl-5,6,7,8-tetrahydrobenzimidazol-2-yl)-1H-benzimidazol-1-yl]-methyl]-biphenyl-2-carbonsäure,
(c) 4'-[[2-n-Propyl-4-methyl-6-(1-methyl-5,6,7,8-tetrahydrobenzimidazol-2-yl)-1H-Benzimidazol-1-yl]-methyl]-2-(1H-tetrazol-5-yl)biphenyl,
(d) 4'-[[2-n-Propyl-4-methyl-6-(1-benzyl-5,6,7,8-tetrahydrobenzimidazol-2-yl)-1H-benzimidazol-1-yl]-methyl]-biphenyl-2-carbonsäure,
(e) 4'-[[2-Ethyl-4-methyl-6-(1-phenyl-5,6,7,8-tetrahydrobenzimidazol-2-yl)-1H-benzimidazol-1-yl]-methyl]-biphenyl-2-carbonsäure,
(f) 4'-[[2-n-Propyl-4-methyl-6-(1-carboxymethyl-5,6,7,8-tetrahydrobenzimidazol-2-yl)-1H-benzimidazol-1-yl]-methyl]-biphenyl-2-carbonsäure,
(g) 4'-[[2-Ethyl-4-methyl-6-(1-ethyl-5,6,7,8-tetrahydro-benzimidazol-2-yl)-1H-benzimidazol-1-yl]-methyl]-2-(1H-tetrazol-5-yl)-biphenyl,
(h) 4'-[[2-n-Propyl-4-methyl-6-(4,4-dimethyl-oxazolin-2-yl)-1H-benzimidazol-1-yl]-methyl]-biphenyl-2-carbonsäure,
(i) 4'-[[2-n-Propyl-4-methyl-6-(1,5-dimethyl-4-phenyl-imidazol-2-yl)-1H-benzimidazol-1-yl]-methyl]-biphenyl-2-carbonsäure und
(k) 4'-[[2-n-Propyl-4-methyl-6-(4-isopropyl-1,5-dimethyl-imidazol-2-yl)-1H-benzimidazol-1-yl]-methyl]-biphenyl-2-carbonsäure

und deren Salze.

Erfindungsgemäß erhält man die neuen Verbindungen nach folgenden Verfahren:

a) Zur Herstellung von Verbindungen der allgemeinen Formel I, in der $R_2$ eine Oxazol-2-yl-, Thiazol-2-yl- oder Imidazol-2-yl-Gruppe darstellt, in denen jeweils über die 4,5-Positionen eine n-Butylenbrücke angefügt ist und zusätzlich die Iminogruppe im Imidazolring durch eine Alkylgruppe mit 1 bis 6 Kohlenstoffatomen, durch eine Phenylalkylgruppe mit 1 bis 3 Kohlenstoffatomen im Alkylteil oder durch eine Phenylgruppe substituiert sein kann:
Umsetzung einer Verbindung der allgemeinen Formel

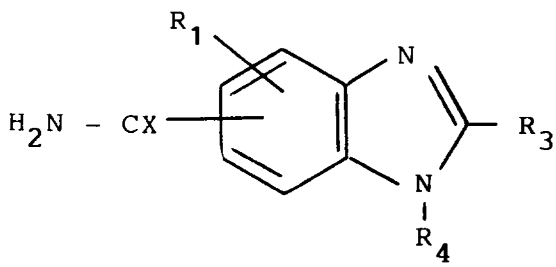

, (II)

in der
$R_1$, $R_3$ und $R_4$ wie eingangs definiert sind und
X ein Sauerstoff- oder Schwefelatom oder eine gegebenenfalls durch eine Alkylgruppe mit 1 bis 6 Kohlenstoffatomen, durch eine Phenylalkylgruppe mit 1 bis 3 Kohlenstoffatomen im Alkylteil oder durch eine Phenylgruppe substituierte Iminogruppe darstellt, mit einem α-Halogenketon der allgemeinen Formel

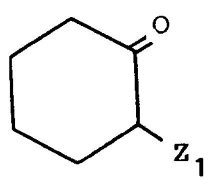

, (III)

in der
$Z_1$ ein Halogenatom wie ein Chloratom darstellt.

Die Umsetzung wird vorzugsweise in Gegenwart eines geeigneten Lösungsmittels wie Dimethylformamid, Diethylenglykoldimethylether, Triethylenglykoldimethylether oder Sulfolan gegebenenfalls in Gegenwart einer Base wie Kaliumkarbonat, Pyridin, Triethylamin, N-Ethyl-diisopropylamin oder N-Ethyl-dicyclohexylamin bei Temperaturen zwischen 0 und 250 °C durchgeführt.

Bedeutet X ein Sauerstoff- oder Schwefelatom, so wird die Umsetzung vorzugsweise in einem Lösungsmittel mit einem Siedepunkt von oberhalb 150 °C oder in der Schmelze bei Temperaturen zwischen 150 und 250 °C, vorzugsweise bei Temperaturen zwischen 175 und 225 °C, durchgeführt.

Bedeutet X eine gegebenenfalls durch eine Alkylgruppe substituierte Iminogruppe so wird die Umsetzung vorzugsweise in Gegenwart eines entsprechenden Amins als Lösungsmittel, z. B. in Gegenwart von flüssigem Ammoniak, Methylamin, Ethylamin, n-Propylamin oder Isopropylamin bei Temperaturen zwischen 0 und 100 °C, vorzugsweise bei Temperaturen zwischen 20 und 75 °C, durchgeführt.

b) Zur Herstellung von Verbindungen der allgemeinen Formel I, in der $R_2$ eine der eingangs erwähnten Imidazol-2-yl-Gruppen darstellt, die in 1-Stellung durch den Rest $R_a$ substituiert sein können:
Umsetzung einer Verbindung der allgemeinen Formel

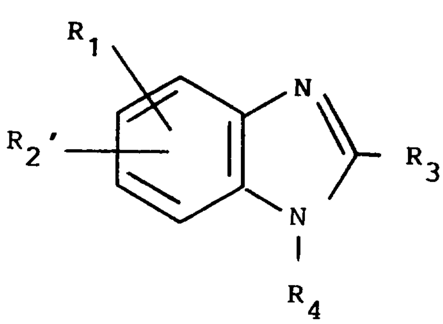

, (IV)

in der
$R_1$, $R_3$ und $R_4$ wie eingangs definiert sind und
$R_2$' eine der eingangs erwähnten Oxazol-2-yl-Gruppen darstellt, mit einem Amin der allgemeinen Formel

$H_2N - R_6$ , (V)

in der
$R_6$ die für $R_a$ eingangs erwähnten Bedeutungen besitzt oder ein Wasserstoffatom darstellt.

Die Umsetzung wird zweckmäßigerweise in einem Überschuß des eingesetzten Amins und vorzugsweise in Gegenwart eines entsprechenden Formamids der Formel $HCONHR_6$ als Lösungsmittel gegebenenfalls in einem Druckgefäß bei erhöhten Temperaturen, z. B. bei Temperaturen zwischen 100 und 250 °C, vorzugsweise bei Temperaturen zwischen 175 und 225 °C, durchgeführt.

Bei der Umsetzung wird gleichzeitig eine gegebenenfalls im Rest $R_4$ vorhandene substituierte Carboxygruppe in die Carboxygruppe oder eine gegebenenfalls vorhandene substituierte Tetrazolylgruppe in die 1H-Tetrazol-5-yl-gruppe übergeführt.

c) Zur Herstellung von Benzimidazolen der allgemeinen Formel I, in der $R_4$ eine Gruppe der Formel

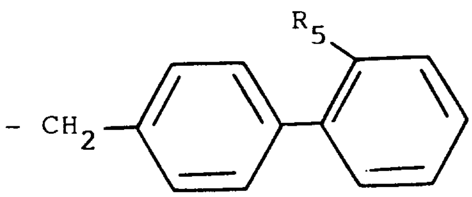

darstellt:
Umsetzung eines Benzimidazols der allgemeinen Formel

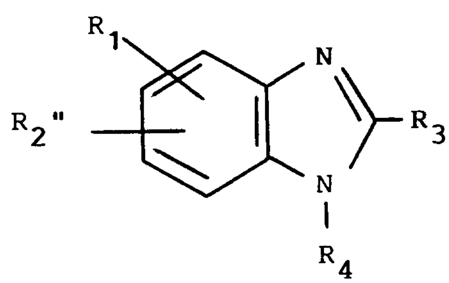

, (VI)

in der
$R_1$, $R_3$ und $R_4$ wie eingangs definiert sind und
$R_2$" mit Ausnahme der in 1-Stellung unsubstituierten Imidazol-2-yl- und Imidazolin-2-yl-Gruppe die für $R_2$ eingangs erwähnten Bedeutungen besitzt, mit einer Biphenylverbindung der allgemeinen Formel

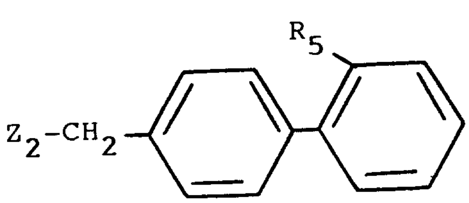

, (VII)

in der
$R_5$ wie eingangs definiert ist und
$Z_2$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch

als Lösungsmittel verwendet werden können, vorzugsweise bei Temperaturen zwischen 0 und 100 °C, z.B. bei Temperaturen zwischen Raumtemperatur und 50 °C, durchgeführt.

Bei der Umsetzung erhält man vorzugsweise ein Gemisch der 1- und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

d) Zur Herstellung einer Verbindung der allgemeinen Formel I, in der $R_5$ eine Carboxygruppe darstellt: Überführung einer Verbindung der allgemeinen Formel

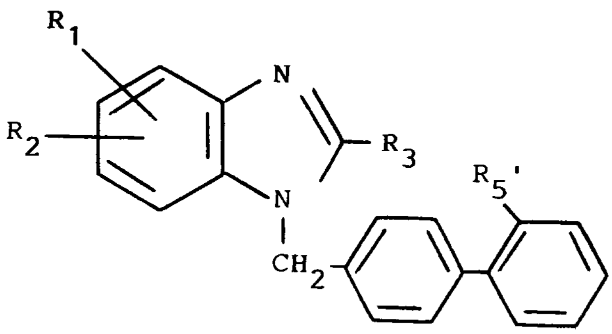

, (VIII)

in der

$R_1$ bis $R_3$ wie eingangs definiert sind und

$R_5$' eine mittels Hydrolyse, Thermolyse oder Hydrogenolyse in eine Carboxygruppe überführbare Gruppe darstellt, in eine Verbindung der allgemeinen Formel I, in der $R_5$ eine Carboxygruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird in Gegenwart einer Säure wie Salzsäure, Bromwasserstoffsäure, Schwefelsäure, Phosphorsäure, Trichloressigsäure oder Trifluoressigsäure gegebenenfalls in Gegenwart eines Reaktionsbeschleunigers wie Hexadecyl-tributylphosphoniumbromid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bedeutet $R_5$' in einer Verbindung der allgemeinen Formel VIII eine Cyano- oder Aminocarbonylgruppe, so können diese Gruppen auch mit einem Nitrit, z.B. Natriumnitrit, in Gegenwart einer Saure wie Schwefelsäure, wobei diese zweckmäßigerweise gleichzeitig als Lösungsmittel verwendet wird, bei Temperaturen zwischen O und 50 °C in die Carboxygruppe übergeführt werden.

Bedeutet $R_5$' in einer Verbindung der allgemeinen Formel VIII beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie Trifluoressigsäure, p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet $R_5$' in einer Verbindung der allgemeinen Formel VIII beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

e) Zur Herstellung von Verbindungen der allgemeinen Formel I, in der $R_2$ eine 5,5-Spiro-cyclopentano-dihydro-imidazol-4-on-2-yl-Gruppe darstellt:

Behandlung eines Benzimidazols der allgemeinen Formel

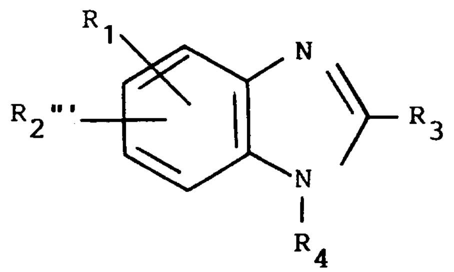

, (IX)

in der

$R_1$, $R_3$ und $R_4$ wie eingangs definiert sind und

$R_2'''$ eine Imidazol-2-yl-Gruppe darstellt, in der über die 4,5-Positionen eine n-Butylenbrücke angefügt ist, mit einer Base in Gegenwart von Luft und Licht.

Die Umsetzung wird in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bei der Umsetzung wird gleichzeitig eine gegebenenfalls im Rest $R_4$ vorhandene Estergruppe in eine Carboxygruppe übergeführt.

f) Zur Herstellung einer Verbindung der allgemeinen Formel I, in der $R_5$ eine 1H-Tetrazolylgruppe darstellt:

Abspaltung eines Schutzrestes von einer Verbindung der allgemeinen Formel

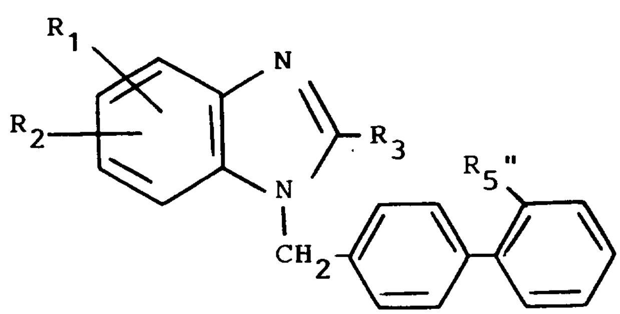

, (X)

in der

$R_1$, $R_2$ und $R_3$ wie eingangs definiert sind und

$R_5''$ eine in 1- oder 2-Stellung durch einen Schutzrest geschützte 1H-Tetrazolyl- oder 2H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 °C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 °C, vorzugsweise bei Temperaturen zwischen 120 und 140 °C.

g) Zur Herstellung einer Verbindung der allgemeinen Formel I, in der $R_5$ eine 1H-Tetrazolylgruppe darstellt:

Umsetzung einer Verbindung der allgemeinen Formel

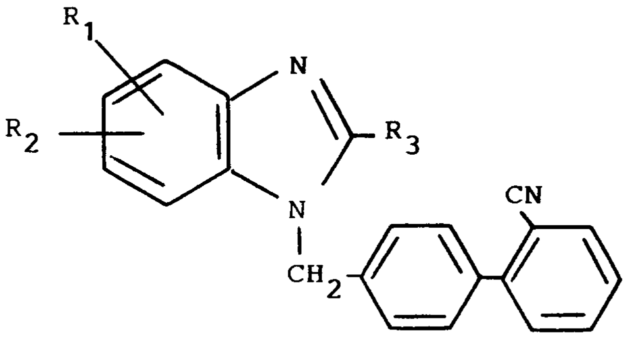

, (XI)

in der
$R_1$ bis $R_3$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 °C, vorzugsweise bei 125 °C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

h) Zur Herstellung von Verbindungen der allgemeinen Formel I, in der $R_2$ eine der eingangs erwähnten Imidazol-2-yl-Gruppen darstellt, die in 1-Stellung durch eine Phenylalkylgruppe, wobei der Phenylkern durch Alkyl-, Hydroxy- oder Alkoxygruppen mono- oder disubstituiert sein kann und die Substituenten gleich oder verschieden sein können, oder durch eine Alkylgruppe mit 1 bis 6 Kohlenstoffatomen, wobei die Alkylgruppe zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, oder eine der eingangs erwähnten Imidazolium-2-yl-Gruppen darstellt:

Umsetzung einer Verbindung der allgemeinen Formel

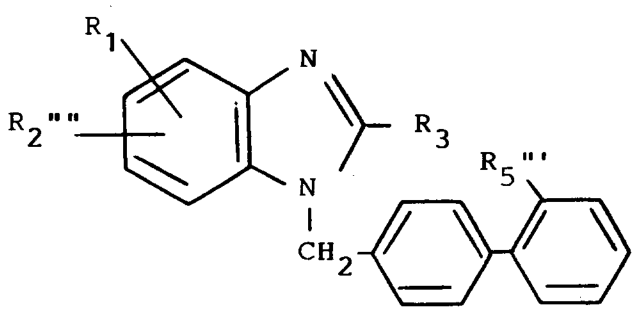

, (XII)

in der
$R_1$ und $R_3$ wie eingangs definiert sind,
$R_2''''$ eine der in 1-Stellung eingangs erwähnten unsubstituierten Imidazol-2-yl-Gruppe und
$R_5'''$ eine Carboxygruppe oder eine mittels Hydrolyse, Thermolyse oder Hydrogenolyse in eine Carboxygruppe überführbare Gruppe oder eine durch einen Schutzrest geschützte 1H-Tetrazolyl- oder 2H-Tetrazolylgruppe darstellen, mit einer Verbindung der allgemeinen Formel

$Z_3$ - $R_7$ , (XIII)

in der
$R_7$ eine Phenylalkylgruppe, wobei der Phenylkern durch Alkyl-, Hydroxy- oder Alkoxygruppen mono- oder disubstituiert sein kann und die Substituenten gleich oder verschieden sein können, oder eine Alkylgruppe mit 1 bis 6 Kohlenstoffatomen, wobei die Alkylgruppe zusätzlich durch eine in vivo in eine

Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, und
$Z_3$ eine nukleophile Austrittsgruppe wie ein Halogenatom,
z. B. ein Chlor-, Brom- oder Jodatom, bedeuten, und erforderlichenfalls anschließende Abspaltung der verwendeten Schutzreste.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Natriumhydrid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

Wird die Umsetzung in Gegenwart eines Überschuß der eingesetzten Verbindung der Formel XIII durchgeführt, so erhält man gleichzeitig eine entsprechende Imidazolium-2-yl-Verbindung der allgemeinen Formel I.

Die anschließende Abspaltung eines Schutzrestes erfolgt vorzugsweise hydrolytisch, thermolytisch oder hydrogenolytisch.

Die hydrolytische Abspaltung eines verwendeten Schutzrestes erfolgt vorzugsweise in Gegenwart einer Säure wie Salzsäure, Bromwasserstoffsäure, Schwefelsäure, Phosphorsäure, Trichloressigsäure oder Trifluoressigsäure gegebenenfalls in Gegenwart eines Reaktionsbeschleunigers wie Hexadecyltributylphosphoniumbromid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches.

Die thermolytische Abspaltung eines Schutzrestes wie des tert.Butyloxycarbonylrestes erfolgt zweckmäßigerweise in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C.

Die hydrogenolytische Abspaltung eines Schutzrestes wie des Benzyloxycarbonylrestes erfolgt in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar.

i) Zur Herstellung von Verbindungen der allgemeinen Formel I, in der $R_2$ einen der durch die Reste $R_8$ bis $R_{10}$ substituierten eingangs erwähnten Imidazol-2-yl-Reste darstellt, wobei jedoch $R_9$ oder $R_{10}$ ein Wasserstoffatom darstellen muß:
Umsetzung eines Aminoketons der allgemeinen Formel

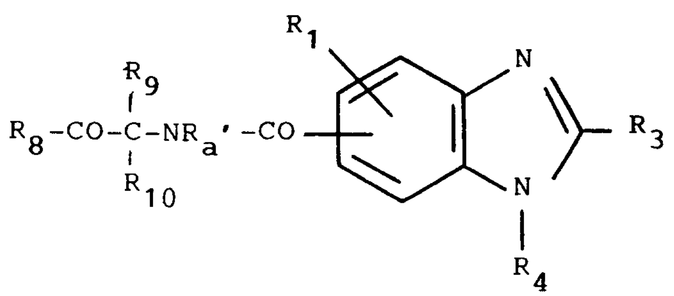

,(XIV)

in der
$R_1$, $R_3$, $R_4$ und $R_8$ bis $R_{10}$ wie eingangs definiert sind, wobei jedoch $R_9$ oder $R_{10}$ ein Wasserstoffatom darstellen muß, und $R_a$' die für $R_a$ eingangs erwähnten Bedeutungen besitzt und ein Wasserstoffatom darstellt, mit einem Ammoniumsalz einer niederen aliphatischen Carbonsäure.

Die Umsetzung wird mit einem Ammoniumsalz einer niederen aliphatischen Carbonsäure wie Ammoniumacetat oder Ammoniumpropionat vorzugsweise in Gegenwart eines Lösungsmittels wie Eisessig oder Propionsäure bei erhöhten Temperaturen, vorzugsweise jedoch bei der Siedetemperatur des Reaktionsgemisches, z.B. bei Temperaturen zwischen 100 und 150 °C, durchgeführt.

j) Zur Herstellung von Verbindungen der allgemeinen Formel I, in der $R_2$ eine der eingangs erwähnten Oxazolin-2-yl- oder Imidazolin-2-yl-Gruppen darstellt:

Dehydratisierung einer Verbindung der allgemeinen Formel

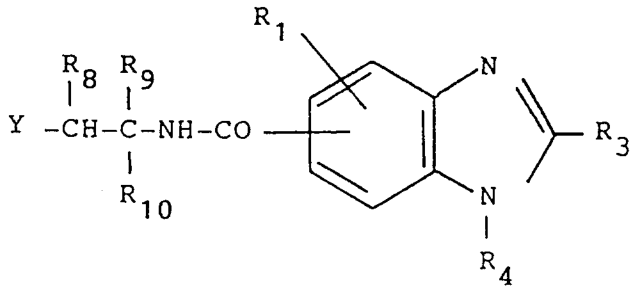

,(XV)

in der

$R_1$, $R_3$, $R_4$ und $R_8$ bis $R_{10}$ wie eingangs definiert sind und

Y eine Hydroxy- oder $HNR_a$-Gruppe darstellt, wobei $R_a$ wie eingangs erwähnt definiert ist.

Die Dehydratisierung wird in Gegenwart eines wasserentziehenden Mittels wie Phosphoroxychlorid, Schwefelsäure, Polyphosphorsäure oder Thionylchlorid, wobei dieses vorzugsweise gleichzeitig auch als Lösungsmittel verwendet wird, bei erhöhten Temperaturen, z.B. bei der Siedetemperatur des verwendeten Dehydratisierungsmittels, z.B. bei Temperaturen zwischen 105 und 150°C, durchgeführt.

k) Zur Herstellung von Verbindungen der allgemeinen Formel I, in der $R_2$ einen der eingangs erwähnten Imidazolin-2-yl-Reste darstellt:

Umsetzung einer Verbindung der allgemeinen Formel

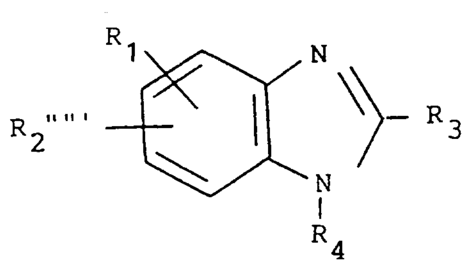

,(XVI)

in der

$R_1$, $R_3$ und $R_4$ wie eingangs definiert sind und

$R_2$''''' einen der durch die Reste $R_8$ bis $R_{10}$ substituierten eingangs für $R_2$ erwähnten Oxazolin-2-yl-Reste darstellt, mit einem Amin der allgemeinen Formel

$H_2N\text{-}(R_8CH)\text{-}(R_9CR_{10})\text{-}NH_2$ ,(XVII)

in der

$R_8$ bis $R_{10}$ wie eingangs definiert sind.

Die Umsetzung wird zweckmäßigerweise in einem Lösungsmittel wie Toluol, Dimethylformamid oder Dimethylsulfoxid, vorzugsweise jedoch in einem Überschuß des eingesetzten Amins der allgemeinen Formel XVII bei erhöhten Temperaturen, z.B. bei Temperaturen zwischen 100 und 150°C, durchgeführt. Die Umsetzung wird jedoch vorzugsweise ohne Lösungsmittel durchgeführt.

1) Zur Herstellung von Verbindungen der allgemeinen Formel I, in der $R_2$ einen der durch die Reste $R_8$ bis $R_{10}$ substituierten eingangs erwähnten Oxazol-2-yl-Reste darstellt:

Dehydratisierung eines Aminoketons der allgemeinen Formel

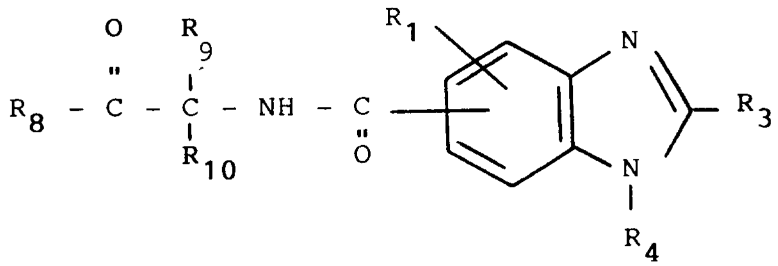

, (XVIII)

in der
$R_1$, $R_3$, $R_4$ und $R_8$ bis $R_{10}$ wie eingangs definiert sind,
wobei jedoch $R_9$ oder $R_{10}$ ein Wasserstoffatom darstellen muß.

Die Dehydratisierung wird in Gegenwart eines wasserentziehenden Mittels wie Phosphoroxychlorid, Phosphorsäure oder Schwefelsäure, wobei dieses vorzugsweise gleichzeitig als Lösungsmittel verwendet wird, bei erhöhten Temperaturen, z.B. bei der Siedetemperatur des verwendeten Dehydratisierungsmittels` z.B. bei Temperaturen zwischen 105 und 150 °C, durchgeführt. Bei der Umsetzung mit Phosphoroxychlorid kann ein gleichzeitig vorhandener tert.Butylester gespalten werden.

m) Zur Herstellung von Verbindungen der allgemeinen Formel I, in der $R_2$ einen der durch die Reste $R_8$ bis $R_{10}$ substituierten eingangs erwähnten Imidazol-2-yl-Reste darstellt:
Dehydrierung einer Verbindung der allgemeinen Formel

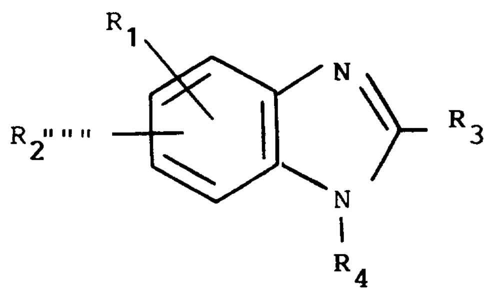

, (XIX)

in der
$R_1$, $R_3$ und $R_4$ wie eingangs definiert sind und
$R_2$''''' einen der durch die Reste $R_8$ bis $R_{10}$ substituierten eingangs für $R_2$ erwähnten Imidazolin-2-yl-Reste darstellt, wobei jedoch $R_9$ oder $R_{10}$ ein Wasserstoffatom darstellen muß.

Die Dehydrierung wird in Gegenwart eines Dehydrierungsmittels wie Palladium/Kohle, Bariummanganat oder Selendioxid, in einem geeigneten Lösungsmittel wie Toluol oder Methylenchlorid bei erhöhten Temperaturen, zum Beispiel bei der Siedetemperatur des verwendeten Lösungsmittels, z.B. Bei Temperaturen zwischen 110 und 150 °C, durchgeführt.

n) Zur Herstellung von Verbindungen der allgemeinen Formel I, in der $R_5$ eine 2,5-Dihydro-5-oxo-1,2,4-oxadiazol-3-yl-Gruppe darstellt:
Umsetzung eines gegebenenfalls im Reaktionsgemisch erhaltenes Amidoxim der allgemeinen Formel

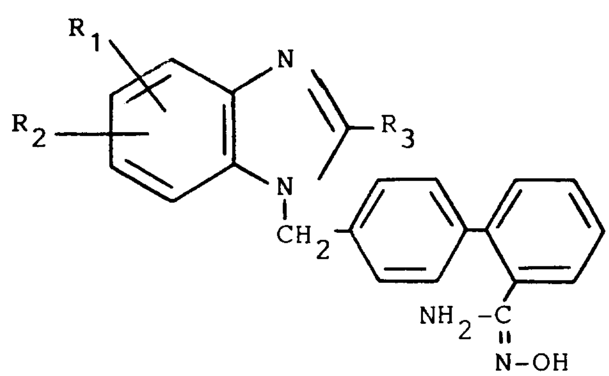

, (XX)

in der
$R_1$ bis $R_3$ wie eingangs definiert sind, mit einer Verbindung der allgemeinen Formel

$Z_4$ - CO - $OR_{11}$ ,(XXI)

in der
$Z_3$ eine nukleophile Austrittsgruppe wie ein Halogenatom,
z.B. ein Chlor-, Brom- oder Jodatom, und
$R_{11}$ eine Alkyl-, Aryl- oder Aralkylgruppe, vorzugsweise eine niedere Alkylgruppe wie die Methyl-, Ethyl-, n-Propyl- oder Isopropylgruppe, bedeuten und anschließende Cyclisierung eines so erhaltenen acylierten Amidoxim.

Die Umsetzung wird zweckmäßigerweise in einem Lösungsmittel wie Methylenchlorid, Chloroform, Tetrahydrofuran, Dioxan oder Acetonitril vorzugsweise in Gegenwart einer anorganischen Base wie Natrium- oder Kaliumcarbonat oder einer organischen Base wie Triethylamin oder Pyridin, wobei die letzteren gleichzeitig auch als Lösungsmittel verwendet werden können, bei Temperaturen zwischen 0 und 20 °C durchgeführt.

Die anschließende Cyclisierung eines so erhaltenen acylierten Amidoxims wird zweckmäßigerweise in einem organischen Lösungsmittel wie Benzol, Toluol, Xylol, Tetrahydrofuran oder Dioxan bei erhöhten Temperaturen, z.B. bei Temperaturen zwischen 50 und 100 °C, vorzugsweise bei der Siedetemperatur des verwendeten Lösungsmittels, durchgeführt. Das hierfür erforderliche Amidoxim erhält man zweckmäßigerweise durch Umsetzung eines entsprechenden Nitrils der allgemeinen Formel XI mit Hydroxylamin in Gegenwart eines Lösungsmittels wie Methanol, Ethanol, Methylenchlorid, Chloroform, Dimethylformamid, Tetrahydrofuran oder Dioxan in Gegenwart einer geeigneten Base wie Natriumcarbonat, Kaliumcarbonat, Natriumhydroxid, Triethylamin, Natriummethylat, Natriumethylat oder Natriumhydrid bei Temperaturen zwischen 50 und 100 °C durchgeführt.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XXI sind teilweise literaturbekannt oder man erhält diese nach literaturbekannten Verfahren.

So erhält man beispielsweise eine Verbindung der allgemeinen Formeln II, IV, VI, VIII, IX, X, XI oder XII durch Acylierung eines entsprechenden o-Phenylendiamins und anschließender Cyclisierung oder

durch Acylierung einer entsprechenden o-Amino-nitroverbindung, anschließender Reduktion der Nitrogruppe und Cyclisierung, wobei ein so gegebenenfalls erhaltenes NH-Benzimidazol mittels Alkylierung mit einem entsprechenden Biphenylderivat in eine in 1-Stellung entsprechend substituierte Verbindung übergeführt werden kann, und gegebenenfalls anschließende Abspaltung eines verwendeten Schutzrestes.

Die in dieser Anmeldung beschriebenen Umwandlungen von Oxazol-2-yl-Verbindungender allgemeinen Formel IV in in 1-Stellung substituierte Imidazol-2-yl-Verbindungen werden analog der in Angew. Chem. 71, 761 (1959) beschriebenen 1H-Imidazolsynthese (Umwandlung von Oxazolen in 1H-Imidazole mit Hilfe von Formamid/Ammoniak) durchgeführt.

Die Oxazole der allgemeinen Formeln IV, VI, VIII, X und XI lassen sich durch Acylierung eines entsprechenden $\alpha$-Aminoketons mit einem entsprechenden Carbonsäurechlorid oder Carbonsäureanhydrid und anschließender Cyclisierung analog J. Chem. Soc. 95, 2167 (1909) und Synthesis 1970, 648, herstellen, wobei als Kondensationsmittel starke Säuren wie z. B. Schwefelsäure, Phosphorsäure, Fluorwasserstoffsäure oder auch $POCl_3$ verwendet werden können.

Eine Ausgangsverbindung der allgemeinen Formel XV erhält man durch Acylierung eines entsprechenden $\beta$-Amino-alkohols und eine Verbindung der allgemeinen Formel XVI durch Cyclisierung einer so erhaltenen Verbindung der allgemeinen Formel XV.

Die vorstehend erwähnten acylierten $\alpha$-Aminoketone lassen sich auch durch Behandlung mit Ammoniumacetat in Eisessig analog Chem. Ber. 106, 2415 (1973) direkt in die entsprechenden substituierten Imidazole überführen.

Eine Ausgangsverbindung der allgemeinen Formeln XIV und XVIII erhält man durch Acylierung eines entsprechenden $\alpha$-Aminoacetons mit einer entsprechenden aktivierten Benzimidazolcarbonsäure, wobei das erforderliche $\alpha$-Amino-aceton nach Dakin/West (siehe Chem. Soc. Rev. 17, 91 (1988)) aus den entsprechenden $\alpha$-Aminosäuren erhalten wird.

Eine Ausgangverbindung der allgemeinen Formel XIX erhält man durch Acylierung eines entsprechenden $\beta$-Amino-alkohols mit einer entsprechenden aktivierten Benzimidazolcarbonsäure, wobei ein so erhaltene Verbindung anschließend cyclisiert und anschließend mit einem entsprechenden Ethylendiamin umgesetzt wird.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[[2-n-Propyl-4-methyl-6-(5,6,7,8-tetrahydrobenzimidazol-2-yl)-1H-benzimidazol-1-yl]-methyl]-biphenyl-2-carbonsäure,

B = 4'-[[2-n-Propyl-4-methyl-6-(1-methyl-5,6,7,8-tetrahydrobenzimidazol-2-yl)-1H-benzimidazol-1-yl]-methyl]-biphenyl-2-carbonsäure,

C = 4'-[[2-n-Propyl-4-methyl-6-(1-methyl-5,6,7,8-tetrahydrobenzimidazol-2-yl)-1H-benzimidazol-1-yl]-methyl]-2-(1H-tetrazol-5-yl)biphenyl,

D = 4'-[[2-n-Propyl-4-methyl-6-(1-benzyl-5,6,7,8-tetrahydrobenzimidazol-2-yl)-1H-benzimidazol-1-yl]-methyl]-biphenyl-2-carbonsäure,

E = 4'-[[2-Ethyl-4-methyl-6-(1-phenyl-5,6,7,8-tetrahydrobenzimidazol-2-yl)-1H-benzimidazol-1-yl]-methyl]-biphenyl-2-carbonsäure-hydrat,

F = 4'-[[2-n-Propyl-4-methyl-6-(1-carboxymethyl-5,6,7,8-tetrahydrobenzimidazol-2-yl)-1H-benzimidazol-1-yl]-methyl]-biphenyl-2-carbonsäure-semihydrat,

G = 4'-[[2-Ethyl-4-methyl-6-(1-ethyl-5,6,7,8-tetrahydro-benzimidazol-2-yl)-1H-benzimidazol-1-yl]-methyl]-2-(1H-tetrazol-5-yl)-biphenyl-hydrat,

H = 4'-[[2-n-Propyl-4-methyl-6-(4,4-dimethyl-oxazolin-2-yl)-1H-benzimidazol-1-yl]-methyl]-biphenyl-2-carbonsäure-di-trifluoracetat,

I = 4'-[[2-n-Propyl-4-methyl-6-(1,5-dimethyl-4-phenyl-imidazol-2-yl)-1H-benzimidazol-1-yl]-methyl]-biphenyl-2-carbonsäure-hydrat und

K = 4'-[[2-n-Propyl-4-methyl-6-(4-isopropyl-1,5-dimethyl-imidazol-2-yl)-1H-benzimidazol-1-yl]-methyl]-biphenyl-2-carbonsäure x 1,25 Wasser

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37 °C mit 50 pM [$^{125}$I]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis K zeigen in dem beschriebenen Test folgende $IC_{50}$-Werte:

| Substanz | $IC_{50}$ [nM] |
|---|---|
| A | 12,0 |
| B | 3,8 |
| C | 2,6 |
| D | 6,0 |
| E | 46,0 |
| F | 38,0 |
| G | 1,6 |
| H | 37,0 |
| I | 3,2 |
| K | 19,5 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'-schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung,

also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel 1

4'-[[2-n-Propyl-4-methyl-6-(5,6,7,8-tetrahydrobenzimidazol-2-yl)-1H-benzimidazol-1-yl]-methyl]-biphenyl-2-carbonsäure-methylester

a) 4'-[(2-n-Propyl-4-methyl-6-amidino-1H-benzimidazol-1-yl)-methyl]-biphenyl-2-carbonsäure-methylester

In eine Lösung von 6,2 g (14,6 mMol) 4'-[[2-n-Propyl-4-methyl-6-cyano-1H-benzimidazol-1-yl]-methyl]-biphenyl-2-carbonsäuremethylester in 750 ml absolutem Methanol wird 3 Stunden bei Raumtemperatur Chlorwasserstoff-Gas eingeleitet und weitere 2 Stunden bei Raumtemperatur gerührt. Nach Entfernen des Lösungsmittels wird im Vakuum eingedampft, der Rückstand 2 mal je 50 ml Methanol und 50 ml Ether aufgenommen und erneut eingedampft. Danach wird der Rückstand in 750 ml absolutem Methanol gelöst und mit 30 g Ammoniumcarbonat versetzt. Nach 12 Stunden bei Raumtemperatur werden 50 g Kieselgel (Korngröße: 0,06-0,3 mm) zugegeben. Nach Filtration und Eindampfen des Filtrats wird der Rückstand an Kieselgel (Korngröße: 0,032-0,063 mm) chromatographiert, wobei als Elutionsmittel Gemische von Methylenchlorid und Methanol steigender Polarität (9:1, 4:1, 3:1 und 1:1) verwendet werden. Die einheitlichen Fraktionen werden vereinigt und eingedampft.
Ausbeute: 4,3 g (57 % der Theorie),
Schaum, $R_f$-Wert: 0,14 (Kieselgel; Methylenchlorid/Ethanol = 9:1)

b) 4'-[(2-n-Propyl-4-methyl-6-(5,6,7,8-tetrahydrobenzimidazol-2-yl)-1H-benzimidazol-1-yl]-methyl]-biphenyl-2-carbonsäure-methylester

0,5 g (1,0 mMol) 4'-[(2-n-Propyl-4-methyl-6-amidino-1H-benzimidazol-1-yl]-methyl]-biphenyl-2-carbonsäure-methylester, 0,13 g (1,0 mMol) 2-Chlor-cyclohexanon und 10 ml flüssiger Ammoniak werden in der Bombe 15 Stunden auf 60 °C erhitzt. Nach Abkühlung und Abdampfen des Ammoniaks wird der Rückstand in Methanol/Methylenchlorid (2:1) gelöst und an Kieselgel (Korngröße: 0,032-0,063 mm) chromatographiert, wobei als Elutionsmittel Gemische von Methylenchlorid und Ethanol steigender Polarität (19:1 und 9:1) verwendet werden. Die einheitlichen Fraktionen werden vereinigt und eingedampft.
Ausbeute: 0,1 g (19 % der Theorie),
Schaum, $R_f$-Wert: 0,50 (Kieselgel; Methylenchlorid/Ethanol = 9:1)

Beispiel 2

4'-[[2-n-Propyl-4-methyl-6-(5,6,7,8-tetrahydrobenzimidazol-2-yl)-1H-benzimidazol-1-yl]-methyl]-biphenyl-2-carbonsäure

0,1 g (0,2 mMol) 4'-[[2-n-Propyl-4-methyl-6-(5,6,7,8-tetrahydrobenzimidazol-2-yl)-1H-benzimidazol-1-yl]-methyl]-biphenyl-2-carbonsäure-methylester und 10 mg (0,02 mMol) Hexadecyl-tributylphosphoniumbromid werden in 10 ml Bromwasserstoffsäure (48%ig) aufgenommen und 15 Minuten auf 110 °C erhitzt. Nach Abkühlung wird mit 20 ml Ether überschichtet und mit 10 ml Wasser verdünnt. Nach Extraktion wird die organische Phase abgetrennt. Die wässrige Phase wird mit Ammoniak auf pH 7 gebracht, der dabei gebildete Niederschlag wird abgesaugt, mit Wasser gewaschen und in Methylenchlorid/Ethanol (4:1) aufgenommen. Das Lösungsmittel wird abgedampft, der Rückstand mit Ether verrieben und getrocknet. Das Rohprodukt wird an Kieselgel (Korngröße: 0,032-0,063 mm) chromatographiert, wobei als Elutionsmittel Methylenchlorid/Ethanol (9:1) verwendet wird. Die einheitlichen Fraktionen werden vereinigt und eingedampft. Der Rückstand wird mit Ether verrieben und getrocknet.
Ausbeute: 64 mg (64 % der Theorie),
Schmelzpunkt: 231-235 °C (Zers.)
$C_{32}H_{32}N_4O_2$ (504,64)
Massenspektrum: $(M + H)^+ = 505$

Beispiel 3

4,-[[2-n-Propyl-4-methyl-6-(5,5-spiro-cyclopentano)-dihydroimidazol-4-on-2-yl)-1H-benzimidazol-1-yl]-methyl]-biphenyl-2-carbonsäure-semihydrat

0,05 g (0,1 mMol) 4'-[[2-n-Propyl-4-methyl-6-(5,6,7,8-tetrahydrobenzimidazol-2-yl)-1H-benzimidazol-1-yl]-methyl]-biphenyl-2-carbonsäure-methylester werden in 4 ml Ethanol gelöst, mit 2 ml 1 N Natronlauge versetzt und 4 Tage bei Raumtemperatur gerührt. Nach Zugabe von 4 ml Wasser wird mit Eisessig der pH-Wert auf 6 gebracht, der dabei gebildete Niederschlag wird abgesaugt, mit Wasser gewaschen und über Kaliumhydroxid getrocknet. Das Rohprodukt wird an Kieselgel (Korngröße: 0,032-0,063 mm) chromatographiert, wobei als Elutionsmittel Methylenchlorid/Ethanol/Eisessig (50:1:0,1 und 30:1:0,1) verwendet wird. Die einheitlichen Fraktionen werden vereinigt und eingedampft. Der Rückstand wird mit Ether verrieben und getrocknet.
Ausbeute: 30 mg (59 % der Theorie),
Schmelzpunkt: 310-311 °C (Zers.)
$C_{32}H_{32}N_4O_3$ (520,64)
Massenspektrum: $(M + H)^+ = 521$

Beispiel 4

4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester

a) 2-n-Propyl-4-methyl-6-(5,6,7,8-tetrahydrobenzoxazol-2-yl)-1H-benzimidazol

2,17 g (10 mMol) 2-n-Propyl-4-methyl-6-aminocarbonyl-1H-benzimidazol und 10,25 g (77 mMol) 2-Chlor-cyclohexanon werden eine Stunde auf 190 °C erhitzt. Nach Abkühlung auf Raumtemperatur wird das Reaktionsgemisch mit Ether verrieben und abgesaugt. Der Rückstand wird in Wasser aufgenommen mit konz. Ammoniak versetzt. Anschließend wird mit Methylenchlorid extrahiert, die organische Phase mit Wasser gewaschen, über Magnesiumsulfat getrocknet und eingedampft. Der Rückstand wird an Kieselgel (Korngröße: 0,032-0,063 mm) chromatographiert, wobei als Elutionsmittel Gemische von Methylenchlorid und Ethanol steigender Polarität (50:1, 25:1 und 20:1) verwendet werden. Die einheitlichen Fraktionen werden vereinigt und eingedampft.
Ausbeute: 1,9 g (64 % der Theorie),
Schaum, $R_f$-Wert: 0,20 (Kieselgel; Essigester)

b) 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester

3,3 g (11 mMol) 2-n-Propyl-4-methyl-6-(5,6,7,8-tetrahydrobenzoxazol-2-yl)-1H-benzimidazol werden in 15 ml Dimethylformamid gelöst und bei 5-10 °C portionsweise mit 1,5 g (13,2 mMol) Kalium-tert.butylat versetzt. Nach 15 Minuten bei 5 °C werden 4,6 g (13,2 mMol) 4'-Brommethyl-biphenyl-2-carbonsäure-tert.butylester zugegeben. Nach weiteren 45 Minuten bei 5 °C wird das Reaktionsgemisch in 200 ml Wasser eingerührt. Der gebildete Niederschlag wird abgesaugt, mit Wasser gewaschen und in 200 ml Essigester aufgenommen. Die Lösung wird mi Wasser und mit gesättigter Natriumchloridlösung gewaschen, über Magnesiumsulfat getrocknet und eingedampft. Der Rückstand wird an Kieselgel (Korngröße: 0,032-0,063 mm) chromatographiert, wobei als Elutionsmittel Methylenchlorid/Ethanol (50:1) verwendet wird. Die einheitlichen Fraktionen werden vereinigt und eingedampft.
Ausbeute: 4,8 g (78 % der Theorie),
Schaum, $R_f$-Wert: 0,23 (Kieselgel; Methylenchlorid/Ethanol = 49:1)

Beispiel 5

4'-[[2-n-Propyl-4-methyl-6-(5,6,7,8-tetrahydrobenzimidazol-2-yl)-1H-benzimidazol-1-yl]-methyl]-biphenyl-2-carbonsaure

2,0 g (3,56 mMol) 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester, 16 ml (40 mMol) Formamid und 40 ml Ammoniak (flüssig)

werden in der Bombe 14 Stunden auf 200 °C erhitzt. Nach Abkühlung wird das Reaktionsgemisch mit Wasser verdünnt, der dabei gebildete Niederschlag wird abgesaugt. Das Filtrat wird mit Eisessig auf pH 6 gestellt, der dabei gebildete Niederschlag wird abzentrifugiert und mit Wasser gewaschen. Der Rückstand wird in 50 ml 2 N Salzsäure aufgenommen. Durch Zugabe von konzentriertem Ammoniak wird der pH 6 eingestellt, der dabei gebildete Niederschlag wird abgesaugt, mit Wasser gewaschen und getrocknet.
Ausbeute: 1,3 g (72 % der Theorie),
Schmelzpunkt: ab 235 °C (Zers.)

Beispiel 6

4'-[[2-n-Propyl-4-methyl-6-(1,3-dimethyl-5,6,7,8-tetrahydrobenzimidazoliumiodid-2-yl)-1H-benzimidazol-1-yl]-methyl]-biphenyl-2-carbonsäure

0,85 g (1,7 mMol) 4'-[[2-n-Propyl-4-methyl-6-(5,6,7,8-tetrahydrobenzimidazol-2-yl)-1H-benzimidazol-1-yl]-methyl]-biphenyl-2-carbonsäure werden in 9 ml Dimethylsulfoxid gelöst, bei 5 °C mit 420 mg (3,7 mMol) Kalium-tert.butylat versetzt und 10 Minuten gerührt. Nach Zugabe von 570 mg (4,0 mMol) Methyljodid wird das Reaktionsgemisch 25 Minuten auf 70 °C erhitzt. Nach Abkühlung wird auf Eis gegossen, der gebildete Niederschlag abgesaugt und mit Wasser gewaschen. Der Rückstand wird in 50 ml Ethanol aufgenommen, mit 12 ml 1 N Natronlauge versetzt und 5 Tage bei Raumtemperatur gerührt. Das Lösungsmittel wird im Vakuum abgedampft, der Rückstand wird mit Eis versetzt und mit wässriger Zitronensäure (5 %) angesäuert. Der dabei gebildete Niederschlag wird abgesaugt, mit Wasser gewaschen und getrocknet.
Ausbeute: 470 mg (42 % der Theorie),
Schmelzpunkt: 240-242 °C (Zers.)

| $C_{34}H_{37}N_4O_2I$ (660,61) | | | | | | |
|---|---|---|---|---|---|---|
| Ber.:<br>Gef.: | C | 61,82<br>61,69 | H | 5,65<br>5,88 | N | 8,48<br>8,72 |

Beispiel 7

4'-[[2-n-Propyl-4-methyl-6-(1-methyl-5,6,7,8-tetrahydrobenzimidazol-2-yl)-1H-benzimidazol-1-yl]-methyl]-biphenyl-2-carbonsäure-hydrat

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester und N-Methylformamid/Methylamin.
Ausbeute: 27 % der Theorie,
Schmelzpunkt: 183-186 °C

| $C_{33}H_{34}N_4O_2 \times H_2O$ (536,68) | | | | | | |
|---|---|---|---|---|---|---|
| Ber.:<br>Gef.: | C | 73,85<br>74,22 | H | 6,76<br>6,97 | N | 10,44<br>10,48 |

Beispiel 8

4'-[[2-n-Propyl-4-methyl-6-(5,6,7,8-tetrahydrobenzimidazol-2-yl)-1H-benzimidazol-1-yl]-methyl]-2-(1H-tetrazol-5-yl)biphenyl-semihydrat

a) 4'-[[2-n-Propyl-4-methyl-6-(5,6,7,8-tetrahydrobenzoxazol-2-yl)-1H-benzimidazol-1-yl]-methyl]-2-(1-triphenylmethyltetrazol-5-yl)biphenyl

Hergestellt analog Beispiel 4b aus 2-n-Propyl-4-methyl-6-(5,6,7,8-tetrahydrobenzoxazol-2-yl)-1H-benzimidazol und 4'-Brommethyl-2-(1-triphenyl-methyl-tetrazol-5-yl)biphenyl.
Öl, $R_f$-Wert: 0,67 (Kieselgel; Essigester)

b) 4'-[[2-n-Propyl-4-methyl-6-(5,6,7,8-tetrahydrobenzimidazol-2-yl)-1H-benzimidazol-1-yl]-methyl]-2-(1H-tetrazol-5-yl)biphenyl-semihydrat

Hergestellt analog Beispiel 5 aus 2-n-Propyl-4-methyl-6-(5,6,7,8-tetrahydrobenzoxazol-2-yl)-1H-benzimidazol-1-yl]-methyl]-2-(1-triphenylmethyl-tetrazol-5-yl)biphenyl und Formamid/Ammoniak.
Ausbeute: 70 % der Theorie,
Schmelzpunkt: ab 230 ° C (Zers.)

| $C_{32}H_{32}N_8$ x 0,5 $H_2O$ (537,68) | | | | | | |
|---|---|---|---|---|---|---|
| Ber.:<br>Gef.: | C | 71,48<br>71,43 | H | 6,19<br>6,46 | N | 20,84<br>21,20 |

## Beispiel 9

4'-[[2-n-Propyl-4-methyl-6-(1-methyl-5,6,7,8-tetrahydrobenzimidazol-2-yl)-1H-benzimidazol-1-yl]-methyl]-2-(1H-tetrazol-5-yl)biphenyl-semihydrat

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-2-(1-triphenylmethyl-tetrazol-5-yl)biphenyl und N-Methylformamid/Methylamin.
Ausbeute: 63 % der Theorie,
Schmelzpunkt: ab 240 ° C (Zers.)

| $C_{33}H_{34}N_8$ x 0,5 $H_2O$ (551,71) | | | | | | |
|---|---|---|---|---|---|---|
| Ber.:<br>Gef.: | C | 71,84<br>71,63 | H | 6,40<br>6,45 | N | 20,31<br>20,64 |

## Beispiel 10

4'-[[2-n-Propyl-4-methyl-6-(5,6,7,8-tetrahydrobenzoxazol-2-yl)-1H-benzimidazol-1-yl]-methyl]-biphenyl-2-carbonsäure

1,2 g (24 mMol) 2-n-Propyl-4-methyl-6-(5,6,7,8-tetrahydrobenzoxazol-2-yl)-1H-benzimidazol-1-yl]-methyl]-biphenyl-2-carbonsäure-tert.butylester werden in 25 ml Methylenchlorid gelöst, mit 8,5 ml Trifluoressigsäure versetzt und 3 Stunden bei Raumtemperatur gerührt. Anschließend wird das Lösungsmittel im Vakuum abgedampft, der Rückstand wird mit Eis versetzt und mit konz. Ammoniak alkalisch gestellt. Nach einer Stunde wird durch Zusatz von Zitronensäure der pH-Wert 5 eingestellt. Der dabei gebildete Niederschlag wird abgesaugt, mit Wasser gewaschen und getrocknet. Das Rohprodukt wird an Kieselgel (Korngröße: 0,032-0,063 nm) gereinigt, wobei als Elutionsmittel Essigester verwendet wird. Die einheitlichen Fraktionen werden vereinigt und eingedampft.
Ausbeute: 33 % der Theorie,
Schmelzpunkt: 229-232 ° C (Zers.)
$C_{32}H_{31}N_3O_3$ (505,62)
Massenspektrum: $M^+$ = 505

## Beispiel 11

4'-[[2-Ethyl-4-methyl-6-(1-methyl-5,6,7,8-tetrahydrobenzimidazol-2-yl)-1H-benzimidazol-1-yl]-methyl]-2-(1H-tetrazol-5-yl)-biphenyl-hydrat

a) 2-Ethyl-4-methyl-6-(5,6,7,8-tetrahydrobenzoxazol-2-yl)-1H-benzimidazol

Hergestellt analog Beispiel 4a aus 2-Ethyl-4-methyl-6-aminocarbonyl-1H-benzimidazol und 2-Chlorcyclohexanon.

Ausbeute: 60 % der Theorie,
Öl, $R_f$-Wert: 0,17 (Kieselgel; Essigester)

b) 4'-[[2-Ethyl-4-methyl-6-(5,6,7,8-tetrahydrobenzoxazol-2-yl)-1H-benzimidazol-1-yl]-methyl-2-(1-triphenylmethyltetrazol-5-yl)-biphenyl

Hergestellt analog Beispiel 4b aus 2-Ethyl-4-methyl-6-(5,6,7,8-tetrahydrobenzoxazol-2-yl)-1H-benzimidazol und 4'-Brommethyl-2-(1-triphenylmethyl-tetrazol-5-yl)-biphenyl.
Ausbeute: 63 % der Theorie,
Öl, $R_f$-Wert: 0,69 (Kieselgel; Essigester)

c) 4'-[[2-Ethyl-4-methyl-6-(1-methyl-5,6,7,8-tetrahydrobenzimidazol-2-yl)-1H-benzimidazol-1-yl]-methyl-2-(1H-tetrazol-5-yl)-biphenyl-hydrat

Hergestellt analog Beispiel 5 aus 4'-[[2-Ethyl-4-methyl-6-(5,6,7,8-tetrahydrobenzoxazol-2-yl)-1H-benzimidazol-1-yl]-methyl-2-(2-triphenylmethyl-tetrazol-5-yl)-biphenyl und N-Methyl-formamid/Methylamin.
Ausbeute: 51 % der Theorie,
Schmelzpunkt: ab 180 °C (Zers.)

| $C_{32}H_{32}N_8$ x $H_2O$ (546,69) | | | | | | |
|---|---|---|---|---|---|---|
| Ber. x $H_2O$:<br>Gef.: | C | 70,31<br>70,07 | H | 6,27<br>6,55 | N | 20,50<br>20,60 |

Massenspektrum: $M^+$ = 528

Beispiel 12

4'-[[2-Ethyl-4-methyl-6-(1-methyl-5,6,7,8-tetrahydrobenzimidazol-2-yl)-1H-benzimidazol-1-yl]-methyl]-biphenyl-2-carbonsäure-hydrat

a) 4'-[[2-Ethyl-4-methyl-6-(5,6,7,8-tetrahydrobenzoxazol-2-yl)-1H-benzimidazol-1-yl]-methyl]-biphenyl-2-carbonsäure-tert.butylester

Hergestellt analog Beispiel 4b aus 2-Ethyl-4-methyl-6-(5,6,7,8-tetrahydrobenzoxazol-2-yl)-1H-benzimidazol und 4'-Brommethyl-biphenyl-2-carbonsäure-tert.butylester.
Ausbeute: 77 % der Theorie,
Schmelzpunkt: 160-162 °C

b) 4'-[[2-Ethyl-4-methyl-6-(1-methyl-5,6,7,8-tetrahydrobenzimidazol-2-yl)-1H-benzimidazol-1-yl]-methyl]-biphenyl-2-carbonsäure-hydrat

Hergestellt analog Beispiel 5 aus 4'-[[2-Ethyl-4-methyl-6-(5,6,7,8-tetrahydrobenzoxazol-2-yl)-1H-benzimidazol-1-yl]-methyl]-biphenyl-2-carbonsäure-tert.butylester und N-Methylformamid/Methylamin.
Ausbeute: 34 % der Theorie,
Schmelzpunkt: 292-300 °C (Zers.)

| $C_{32}H_{32}N_4O_2$ x $H_2O$ (522,66) | | | | | | |
|---|---|---|---|---|---|---|
| Ber. x $H_2O$:<br>Gef.: | C | 73,54<br>73,38 | H | 6,56<br>6,76 | N | 10,72<br>10,67 |

Massenspektrum: $M^+$ = 504

Beispiel 13

4'-[[2-Ethyl-4-methyl-6-(1-phenyl-5,6,7,8-tetrahydrobenzimidazol-2-yl)-1H-benzimidazol-1-yl]-methyl]-biphenyl-2-carbonsäure-hydrat

Hergestellt analog Beispiel 5 aus 4'-[[2-Ethyl-4-methyl-6-(5,6,7,8-tetrahydrobenzoxazol-2-yl)-1H-benzimidazol-1-yl]-methyl]-biphenyl-2-carbonsäure-tert.butylester und N-Formylanilid/Anilin.
Ausbeute: 13 % der Theorie,
Schmelzpunkt: 255-257 ° C (Zers.)

| $C_{37}H_{34}N_4O_2$ x $H_2O$ (584,73) | | | | | | |
|---|---|---|---|---|---|---|
| Ber. x $H_2O$:<br>Gef.: | C | 76,00<br>76,36 | H | 6,20<br>6,18 | N | 9,58<br>9,59 |

Massenspektrum: $M^+$ = 566

Beispiel 14

4'-[[2-n-Propyl-4-methyl-6-(1-phenyl-5,6,7,8-tetrahydrobenzimidazol-2-yl)-1H-benzimidazol-1-yl]-methyl]-biphenyl-2-carbonsäure-semihydrat

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester und N-Formylanilid/Anilin.
Ausbeute: 23 % der Theorie,
Schmelzpunkt: 258-260 ° C (Zers.)

| $C_{38}H_{36}N_4O_2$ x 1/2 $H_2O$ (589,75) | | | | | | |
|---|---|---|---|---|---|---|
| Ber. x 1/2 $H_2O$:<br>Gef.: | C | 77,39<br>77,03 | H | 6,32<br>6,30 | N | 9,50<br>9,39 |

Massenspektrum: $M^+$ = 580

Beispiel 15

4'-[[2-n-Propyl-4-methyl-6-(1-benzyl-5,6,7,8-tetrahydrobenzimidazol-2-yl)-1H-benzimidazol-1-yl]-methyl]-biphenyl-2-carbonsäure

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester und N-Benzylformamid/Benzylamin.
Ausbeute: 54 % der Theorie,
Schmelzpunkt: 256-258 ° C (Zers.)

| $C_{39}H_{38}N_4O_2$ (594,77) | | | | | | |
|---|---|---|---|---|---|---|
| Ber.:<br>Gef.: | C | 78,76<br>78,50 | H | 6,44<br>6,49 | N | 9,42<br>9,35 |

Massenspektrum: $M^+$ = 594

Beispiel 16

4'-[[2-n-Propyl-4-methyl-6-(1-ethyl-5,6,7,8-tetrahydrobenzimidazol-2-yl)-1H-benzimidazol-1-yl]-methyl]-biphenyl-2-carbonsäure-sequihydrat

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester und N-Ethylformamid/Ethylamin.
Ausbeute: 17 % der Theorie,
Schmelzpunkt: ab 228 ° C (Zers.)

| $C_{34}H_{36}N_4O_2$ x 1,5 $H_2O$ (559,71) | | | | | | |
|---|---|---|---|---|---|---|
| Ber. x 1,5 $H_2O$:<br>Gef.: | C | 72,96<br>73,04 | H | 7,02<br>6,90 | N | 10,01<br>9,77 |

Massenspektrum: $M^+$ = 532

Beispiel 17

4'-[[2-Ethyl-4-methyl-6-(1-isopropyl-5,6,7,8-tetrahydrobenzimidazol-2-yl)-1H-benzimidazol-1-yl]-methyl]-biphenyl-2-carbonsäure

Hergestellt analog Beispiel 5 aus 4'-[[2-Ethyl-4-methyl-6-(5,6,7,8-tetrahydrobenzoxazol-2-yl)-1H-benzimidazol-1-yl]-methyl]-biphenyl-2-carbonsäure-tert.butylester und N-Isopropyl-formamid/Isopropylamin.
Ausbeute: 2 % der Theorie,
Schmelzpunkt: 197 ° C
$C_{34}H_{36}N_4O_2$ (532,69)
Massenspektrum: $M^+$ = 532

Beispiel 18

4'-[[2-n-Propyl-4-methyl-6-(1-isobutyl-5,6,7,8-tetrahydrobenzimidazol-2-yl)-1H-benzimidazol-1-yl]-methyl]-biphenyl-2-carbonsäure

Hergestellt analog Beispiel 5 aus 4'-[[2-n-Propyl-4-methyl-6-(5,6,7,8-tetrahydrobenzoxazol-2-yl)-1H-benzimidazol-1-yl]-methyl]-biphenyl-2-carbonsäure-tert.butylester und Isobutylamin/Wasser.
Ausbeute: 5 % der Theorie,
$C_{36}H_{40}N_4O_2$ (560,75)
Massenspektrum: $(M+H)^+$ = 561

Beispiel 19

4'-[[2-n-Propyl-4-methyl-6-(1,3-dibenzyl-5,6,7,8-tetrahydrobenzimidazoliumacetat-2-yl)-1H-benzimidazol-1-yl]-methyl]-biphenyl-2-carbonsäure-semihydrat

Hergestellt analog Beispiel 6 aus 4'-[[2-n-Propyl-4-methyl-6-(5,6,7,8-tetrahydrobenzimidazol-2-yl)-1H-benzimidazol-1-yl]-methyl]-biphenyl-2-carbonsäure und Benzylbromid/Natronlauge/Eisessig.
Ausbeute: 76 % der Theorie,
Schmelzpunkt: sintert ab 80 ° C

| $C_{46}H_{44}N_4O_2$ x $CH_3COOH$ x 1/2 $H_2O$ (753,95) | | | | | | |
|---|---|---|---|---|---|---|
| Ber. x $CH_3COOH$ x 1/2 $H_2O$:<br>Gef.: | C | 76,47<br>76,46 | H | 6,55<br>6,65 | N | 7,43<br>7,76 |

Massenspektrum: $M^+$ = 684

Beispiel 20

4'-[[2-n-Propyl-4-methyl-6-(1-carboxymethyl-5,6,7,8-tetrahydrobenzimidazol-2-yl)-1H-benzimidazol-1-yl]-methyl]-biphenyl-2-carbonsäure-semihydrat

Hergestellt analog Beispiel 6 aus 4'-[[2-n-Propyl-4-methyl-6-(5,6,7,8-tetrahydrobenzimidazol-2-yl)-1H-benzimidazol-1-yl]-methyl]-biphenyl-2-carbonsäure und Bromessigsäureethylester/Natronlauge.
Ausbeute: 34 % der Theorie,
Schmelzpunkt: 239-242 ° C

| $C_{34}H_{34}N_4O_4$ x 1/2 $H_2O$ (571,69) | | | | | | |
|---|---|---|---|---|---|---|
| Ber. x 1/2 $H_2O$:<br>Gef.: | C | 71,43<br>71,39 | H | 6,17<br>6,19 | N | 9,80<br>9,81 |

Massenspektrum: $M^+$ = 562

Beispiel 21

4'-[[2-Cyclopropyl-4-methyl-6-(1-methyl-5,6,7,8-tetrahydrobenzimidazol-2-yl)-1H-benzimidazol-1-yl]-methyl]-biphenyl-2-carbonsäure-semihydrat

Hergestellt analog Beispiel 5 aus 4'-[[2-Cyclopropyl-4-methyl-6-(5,6,7,8-tetrahydrobenzoxazol-2-yl)-1H-benzimidazol-1-yl]-methyl]-biphenyl-2-carbonsäure-tert.butylester und N-Methyl-formamid/Methylamin.
Ausbeute: 50 % der Theorie,
Schmelzpunkt: 285-289 ° C

| $C_{33}H_{32}N_4O_2$ x 1/2 $H_2O$ (525,66) | | | | | | |
|---|---|---|---|---|---|---|
| Ber.:<br>Gef.: | C | 75,40<br>75,24 | H | 6,33<br>6,44 | N | 10,66<br>10,42 |

Massenspektrum: $M^+$ = 516

Analog den vorstehenden Beispielen können folgende Verbindungen erhalten werden:

(1) 4'-[[2-Cyclopropyl-4-methyl-6-(1-methyl-5,6,7,8-tetrahydrobenzimidazol-2-yl)-1H-benzimidazol-1-yl]-methyl]-2-(1H-tetrazol-5-yl)biphenyl

(2) 4'-[[2-Ethoxy-4-methyl-6-(1-methyl-5,6,7,8-tetrahydrobenzimidazol-2-yl)-1H-benzimidazol-1-yl]-methyl]-biphenyl-2-carbonsäure

(3) 4'-[[2-Ethoxy-4-methyl-6-(1-methyl-5,6,7,8-tetrahydrobenzimidazol-2-yl)-1H-benzimidazol-1-yl]-methyl]-2-(1H-tetrazol-5-yl)biphenyl

(4) 4'-[[2-Ethyl-4-methyl-6-(1-isopropyl-5,6,7,8-tetrahydrobenzimidazol-2-yl)-1H-benzimidazol-1-yl]-methyl]-2-(1H-tetrazol-5-yl)biphenyl

(5) 4'-[[2-n-Propyl-4-methyl-6-(1-isopropyl-5,6,7,8-tetrahydrobenzimidazol-2-yl)-1H-benzimidazol-1-yl]-methyl]-biphenyl-2-carbonsäure

(6) 4'-[[2-n-Propyl-4-methyl-6-(1-isopropyl-5,6,7,8-tetrahydrobenzimidazol-2-yl)-1H-benzimidazol-1-yl]-methyl]-2-(1H-tetrazol-5-yl)biphenyl

(7) 4'-[[2-Ethyl-4-methyl-6-(1-isobutyl-5,6,7,8-tetrahydrobenzimidazol-2-yl)-1H-benzimidazol-1-yl]-methyl]-biphenyl-2-carbonsäure

(8) 4'-[[2-Ethyl-4-methyl-6-(1-isobutyl-5,6,7,8-tetrahydrobenzimidazol-2-yl)-1H-benzimidazol-1-yl]-methyl]-2-(1H-tetrazol-5-yl)biphenyl

(9) 4'-[[2-n-Propyl-4-methyl-6-(1-isopropyl-5,6,7,8-tetrahydrobenzimidazol-2-yl)-1H-benzimidazol-1-yl]-methyl]-2-(1H-tetrazol-5-yl)biphenyl

(10) 4'-[[2-n-Propyl-4-methyl-6-(1-carboxymethyl-5,6,7,8-tetrahydrobenzimidazol-2-yl)-1H-benzimidazol-1-yl]-methyl]-2-(1H-tetrazol-5-yl)biphenyl

(11) 4'-[[2-n-Propyl-4-methyl-6-(1-methyl-4,5-trimethylenimidazol-2-yl)-1H-benzimidazol-1-yl]-methyl]-biphenyl-2-carbonsäure

(12) 4'-[[2-n-Propyl-4-methyl-6-(1-methyl-4,5-trimethylenimidazol-2-yl)-1H-benzimidazol-1-yl]-methyl]-2-(1H-tetrazol-5-yl)biphenyl

Beispiel 22

4'-[[2-Ethyl-4-methyl-6-(5,6,7,8-tetrahydro-benzoxazol-2-yl)-1H-benzimidazol-1-yl]-methyl]-2-(1H-tetrazol-5-yl)-biphenyl

Hergestellt analog Beispiel 10 aus 4'-[[2-Ethyl-4-methyl-6-(5,6,7,8-tetrahydro-benzoxazol-2-yl)-1H-benzimidazol-1-yl]-methyl]-2-(1-triphenylmethyl-tetrazol-5-yl)-biphenyl und Salzsäure in Methanol.
Ausbeute: 15 % der Theorie,
Schmelzpunkt: 140-142 ° C (Zers.)
$C_{31}H_{29}N_7O$ (515,63)
Massenspektrum: $(M+H)^+ = 516$

Beispiel 23

4'-[[2-Ethyl-4-methyl-6-(1-ethyl-5,6,7,8-tetrahydro-benzimidazol-2-yl)-1H-benzimidazol-1-yl]-methyl]-2-(1H-tetrazol-5-yl)-biphenyl-hydrat

Hergestellt analog Beispiel 5 aus 4'-[[2-Ethyl-4-methyl-6-(5,6,7,8-tetrahydro-benzoxazol-2-yl)-1H-benzimidazol-1-yl]-methyl]-2-(1-triphenylmethyl-tetrazol-5-yl)-biphenyl und N-Ethyl-formamid/Ethylamin.
Ausbeute: 25 % der Theorie,
Schmelzpunkt: ab 180 ° C (Zers.)

| $C_{33}H_{34}N_8$ x $H_2O$ (560,72) | | | | | | |
|---|---|---|---|---|---|---|
| Ber.:<br>Gef.: | C | 70,68<br>70,46 | H | 6,47<br>6,44 | N | 19,99<br>19,56 |

Massenspektrum: $M^+ = 542$

Beispiel 24

4'-[[2-n-Propyl-4-methyl-6-(4,4-dimethyl-oxazolin-2-yl)-1H-benzimidazol-1-yl]-methyl]-biphenyl-2-carbonsaure-di-trifluoracetat

Hergestellt analog Beispiel 10 aus 4'-[[2-n-Propyl-4-methyl-6-(4,4-dimethyl-oxazolin-2-yl)-1H-benzimidazol-1-yl]-methyl]-biphenyl-2-carbonsäure-tert.butylester und Trifluoressigsäure.
Ausbeute: 60 % der Theorie,
Schmelzpunkt: 158-159 ° C

| $C_{30}H_{31}N_3O_3$ x 2 $CF_3COOH$ (709,64) | | | | | | |
|---|---|---|---|---|---|---|
| Ber.:<br>Gef.: | C | 57,55<br>57,76 | H | 4,69<br>4,72 | N | 5,92<br>6,02 |

Massenspektrum: $M^+ = 481$

Beispiel 25

4'-[[2-n-Propyl-4-methyl-6-(1,5-dimethyl-4-phenyl-imidazol-2-yl)-1H-benzimidazol-1-yl]-methyl]-biphenyl-2-carbonsäurehydrat

a) 4'-[[2-n-Propyl-4-methyl-6-[N-(1-benzoyl-ethyl)-methylaminocarbonyl]-1H-benzimidazol-1-yl]-methyl]-biphenyl-2-carbonsäure-tert.butylester

Zu einer Lösung von 1,0 g (2,0 mMol) 4'-[[2-n-Propyl-4-methyl-6-chlorcarbonyl-1H-benzimidazol-1-yl]-methyl]-biphenyl-2-carbonsäure-tert.butylester in 20 ml Methylenchlorid werden 20 ml Toluol und 0,44 g (2,2 mMol) 2-Methylamino-propiophenon zugegeben. Das Reaktionsgemisch wird auf 85 °C erhitzt und innerhalb von 4 Stunden tropfenweise mit 10 ml Pyridin versetzt. Anschließend wird das Reaktionsgemisch eingedampft, der Rückstand mit Eiswasser versetzt und 2 x mit Methylenchlorid extrahiert. Die vereinigten organischen Phasen werden über Magnesiumsulfat getrocknet und eingedampft. Das Rohprodukt wird an Kieselgel (Korngröße: 0,032-0,063 mm) chromatographiert, wobei als Elutionsmittel anfangs Methylenchlorid, später Methylenchlorid/Ethanol/Ammoniak (50:1:0,25 und 25:1:0,01) verwendet wird. Die einheitlichen Fraktionen werden vereinigt und eingedampft.
Ausbeute: 1,0 g (79 % der Theorie),
Schaum, $R_f$-Wert: 0,50 (Kieselgel; Methylenchlorid/Ethanol = 9:1)

b) 4'-[[2-n-Propyl-4-methyl-6-(1,5-dimethyl-4-phenyl-imidazol-2-yl)-1H-benzimidazol-1-yl]-methyl]-biphenyl-2-carbonsäure-tert.butylester

Eine Lösung von 1,0 g (1,5 mMol) 4'-[[2-n-Propyl-4-methyl-6-[N-(1-benzoyl-ethyl)-methylaminocarbonyl]-1H-benzimidazol-1-yl]-methyl]-biphenyl-2-carbonsäure-tert.butylester und 15 g Ammoniumacetat in 80 ml Eisessig wird 2,5 Stunden unter Rückfluß erhitzt. Anschließend wird das Reaktionsgemisch zur Hälfte eingedampft, der Rückstand mit Eiswasser versetzt und 2 x mit Essigester extrahiert. Die vereinigten organischen Phasen werden mit Wasser gewaschen, über Magnesiumsulfat getrocknet und eingedampft. Das Rohprodukt wird an Kieselgel (Korngröße: 0,032-0,063 mm) chromatographiert, wobei als Elutionsmittel Methylenchlorid mit steigenden Mengen Ethanol (3 %, 10 % und 20 %) verwendet wird. Die einheitlichen Fraktionen werden vereinigt und eingedampft.
Ausbeute: 0,68 g (74 % der Theorie),
Schaum, $R_f$-Wert: 0,40 (Kieselgel; Methylenchlorid/Ethanol = 19:1)

c) 4'-[[2-n-Propyl-4-methyl-6-(1,5-dimethyl-4-phenyl-imidazol-2-yl)-1H-benzimidazol-1-yl]-methyl]-biphenyl-2-carbonsäure-hydrat

Hergestellt analog Beispiel 10 aus 4'-[[2-n-Propyl-4-methyl-6-(1,5-dimethyl-4-phenyl-imidazol-2-yl]-methyl]-biphenyl-2-carbonsäure-tert.butylester und Trifluoressigsäure.
Ausbeute: 90 % der Theorie,
Schmelzpunkt: ab 152 °C (Zers.)

| $C_{36}H_{33}N_4O_2$ x $H_2O$ (572,72) | | | | | | |
|---|---|---|---|---|---|---|
| Ber.:<br>Gef.: | C | 75,50<br>75,95 | H | 6,34<br>6,48 | N | 9,78<br>9,92 |

Massenspektrum: $M^+$ = 554

Beispiel 26

4'-[[2-n-Propyl-4-methyl-6-(1-methyl-4,5-diphenyl-imidazol-2-yl)-1H-benzimidazol-1-yl]-methyl]-biphenyl-2-carbonsäuresesquihydrat

a) 4'-[[2-n-propyl-4-methyl-6-(1-methyl-4,5-diphenyl-imidazol-2-yl)-1H-benzimidazol-1-yl]-methyl]-biphenyl-2-carbonsäure-tert.butylester

Hergestellt analog Beispiel 25b aus 4'-[[2-n-Propyl-4-methyl-6-[N-(1-benzoyl-benzyl)-methylaminocarbonyl]-1H-benzimidazol-1-yl]-methyl]-biphenyl-2-carbonsäure-tert.butylester und Ammoniumacetat in Eisessig.
Ausbeute: 27 % der Theorie,
Öl, $R_f$-Wert: 0,40 (Kieselgel; Methylenchlorid/Ethanol = 19:1)

b) 4'-[[2-n-Propyl-4-methyl-6-(1-methyl-4,5-diphenyl-imidazol-2-yl)-1H-benzimidazol-1-yl]-methyl]-biphenyl-2-carbonsäure-sesquihydrat

Hergestellt analog Beispiel 10 aus 4'-[[2-n-Propyl-4-methyl-6-(1-methyl-4,5-diphenyl-imidazol-2-yl)-1H-benzimidazol-1-yl]-methyl]-biphenyl-2-carbonsäure-tert.butylester und Trifluoressigsäure.
Ausbeute: 60 % der Theorie,
Schmelzpunkt: 325-328 °C (Zers.)

| $C_{41}H_{36}N_4O_2$ x 1,5 $H_2O$ (643,79) | | | | | | |
|---|---|---|---|---|---|---|
| Ber.:<br>Gef.: | C | 76,49<br>76,53 | H | 6,11<br>6,15 | N | 8,70<br>8,75 |

Massenspektrum: $M^+$ = 616

Beispiel 27

4'-[[2-n-Propyl-4-methyl-6-(5-methyl-4-isopropyl-imidazol-2-yl)-1H-benzimidazol-1-yl]-methyl]-biphenyl-2-carbonsäuredihydrat-acetat

a) 4'-[[2-n-Propyl-4-methyl-6-(5-methyl-4-isopropyl-imidazol-2-yl)-1H-benzimidazol-1-yl]-methyl]-biphenyl-2-carbonsäure-tert.butylester

Hergestellt analog Beispiel 25b aus 4'-[[2-n-Propyl-4-methyl-6-[N-(1-acetyl-2-methyl-n-propyl)-methylaminocarbonyl]-1H-benzimidazol-1-yl]-methyl]-biphenyl-2-carbonsäure-tert.butylester und Ammoniumacetat in Eisessig.
Ausbeute: 45 % der Theorie,
Öl, $R_f$-Wert: 0,10 (Kieselgel; Essigester/Petrolether = 2:1)

b) 4'-[[2-n-Propyl-4-methyl-6-(5-methyl-4-isopropyl-imidazol-2-yl)-1H-benzimidazol-1-yl]-methyl]-biphenyl-2-carbonsäure-dihydrat-acetat

Hergestellt analog Beispiel 10 aus 4'-[[2-n-Propyl-4-methyl-6-(5-methyl-4-isopropyl-imidazol-2-yl)-1H-benzimidazol-1-yl]-methyl]-biphenyl-2-carbonsäure-tert.butylester und Trifluoressigsäure.
Ausbeute: 75 % der Theorie,
Schmelzpunkt: ab 155 °C (Zers.)

| $C_{32}H_{34}N_4O_2$ x $CH_3COOH$ x 2 $H_2O$ (602,74) | | | | | | |
|---|---|---|---|---|---|---|
| Ber.:<br>Gef.: | C | 67,75<br>67,69 | H | 7,02<br>7,02 | N | 9,30<br>9,53 |

Massenspektrum: $M^+$ = 506

Beispiel 28

4'-[[2-n-Propyl-4-methyl-6-(4-methyl-imidazolin-2-yl)-1H-benzimidazol-1-yl]-methyl]-biphenyl-2-carbonsäure-di-trifluoracetat

a) 4'-[[2-n-Propyl-4-methyl-6-(4-methyl-imidazolin-2-yl)-1H-benzimidazol-1-yl]-methyl]-biphenyl-2-carbonsäure-tert.-butylester

Eine Mischung von 0,43 g (0,8 mMol) 4'-[[2-n-Propyl-4-methyl-6-(4,4-dimethyl-oxazolin-2-yl)-1H-benzimidazol-1-yl]-methyl]-biphenyl-2-carbonsäure-tert.butylester und 0,67 ml (5,8 mMol) 1,2-Diaminopropan werden 48 Stunden auf 120 °C erhitzt. Der nach Abkühlung auf Raumtemperatur erhaltene gelbe Feststoff wird 1 Stunde mit Wasser verrührt, abgesaugt und getrocknet. Das Rohprodukt wird an Kieselgel (Korngröße: 0,032-0,063 mm) chromatographiert, wobei als Elutionsmittel Methylenchlorid/Ethanol/Ammoniak (50:1:0,05, 20:1:0,02 und 7:1:0,07) verwendet wird. Die einheitlichen Fraktionen werden vereinigt und eingedampft.
Ausbeute: 0,26 g (62 % der Theorie),
Schaum, $R_f$-Wert: 0,20 (Kieselgel; Methylenchlorid/Ethanol = 9:1 + Ammoniak)

b) 4'-[[2-n-Propyl-4-methyl-6-(4-methyl-imidazolin-2-yl)-1H-benzimidazol-1-yl]-methyl]-biphenyl-2-carbonsäure-di-trifluoracetat

Hergestellt analog Beispiel 10 aus 4'-[[2-n-Propyl-4-methyl-6-(4-methyl-imidazolin-2-yl)-1H-benzimidazol-1-yl]-methyl]-biphenyl-2-carbonsäure-tert.butylester und Trifluoressigsaure.
Ausbeute: 72 % der Theorie,
Schmelzpunkt: 115-118 °C (Zers., sintert ab 100 °C)

| $C_{29}H_{30}N_4O_2$ x 2 $CF_3COOH$ (694,63) | | | | | | |
|---|---|---|---|---|---|---|
| Ber.:<br>Gef.: | C | 57,06<br>57,02 | H | 4,64<br>5,02 | N | 8,07<br>8,13 |

Massenspektrum: $M^+$ = 466

Beispiel 29

4'-[[2-n-Propyl-4-methyl-6-(4-isopropyl-5-methyl-oxazol-2-yl)-1H-benzimidazol-1-yl]-methyl]-biphenyl-2-carbonsäure

3,9 g (6,7 mMol) 4'-[[2-n-Propyl-4-methyl-6-[N-(1-acetyl-2-methyl-n-propyl)-aminocarbonyl]-1H-benzimidazol-1-yl]-methyl]-biphenyl-2-carbonsäure-tert.butylester werden in 50 ml Phosphoroxychlorid gelöst und 2,5 Stunden bei 105 °C gerührt. Anschließend wird das Phosphoroxychlorid abgezogen, der Rückstand wird mit Wasser bei 80 °C zersetzt und nach Abkühlung mit konz. Ammoniak versetzt. Durch Zugabe von Eisessig wird der pH-Wert auf 5 eingestellt, der dabei gebildete Niederschlag wird abgesaugt, mit Wasser gewaschen, in Methylenchlorid/Methanol (9:1) aufgenommen und über Magnesiumsulfat getrocknet. Das Rohprodukt wird an Kieselgel (Korngröße: 0,032-0,063 mm) chromatographiert, wobei als Elutionsmittel Petrolether/Essigester/Eisessig (1:1:0,002) und Methylenchlorid/Ethanol/Eisessig (20:1:0,002) verwendet werden. Die einheitlichen Fraktionen werden vereinigt, eingedampft, mit Ether verrieben und abgesaugt.
Ausbeute: 2,4 g (71 % der Theorie),
Schmelzpunkt: 222-223 °C

| $C_{32}H_{33}N_3O_3$ (507,64) | | | | | | |
|---|---|---|---|---|---|---|
| Ber.:<br>Gef.: | C | 75,71<br>75,61 | H | 6,55<br>6,59 | N | 8,28<br>8,36 |

Massenspektrum: $M^+$ = 507

Beispiel 30

4'-[[2-n-Propyl-4-methyl-6-(4-isopropyl-1,5-dimethyl-imidazol-2-yl)-1H-benzimidazol-1-yl]-methyl]-biphenyl-2-carbonsäure x 1,25 Wasser

Hergestellt analog Beispiel 5 aus 4'-[[2-n-Propyl-4-methyl-6-(4-isopropyl-5-methyl-oxazol-2-yl)-1H-benzimidazol-1-yl]-methyl]-biphenyl-2-carbonsäure-tert.butylester und N-Methyl-formamid/Methylamin.
Ausbeute: 38 % der Theorie,
Schmelzpunkt: ab 150 °C (Zers.)

| $C_{33}H_{36}N_4O_2$ x 1,25 $H_2O$ (543,20) | | | | | | |
|---|---|---|---|---|---|---|
| Ber.: | C | 72,97 | H | 7,14 | N | 10,32 |
| Gef.: | | 72,95 | | 7,02 | | 9,94 |

Massenspektrum: $M^+$ = 520

Beispiel 31

4'-[[2-n-Propyl-4-methyl-6-(1-ethyl-4-isopropyl-5-methylimidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isopropyl-5-methyl-oxazol-2-yl)-1H-benzimidazol-1-yl]-methyl]-biphenyl-2-carbonsäure-tert.butylester und N-Ethylformamid/Ethylamin.

Beispiel 32

4'-[[2-n-Propyl-4-methyl-6-(1-isopropyl-4-isopropyl-5-methylimidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isopropyl-5-methyl-oxazol-2-yl)-1H-benzimidazol-1-yl]-methyl]-biphenyl-2-carbonsäure-tert.butylester und N-Isopropyl-formamid/Isopropylamin.

Beispiel 33

4'-[[2-n-Propyl-4-methyl-6-(1-cyclohexyl-4-isopropyl-5-methylimidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isopropyl-5-methyl-oxazol-2-yl)-1H-benzimidazol-1-yl]-methyl]-biphenyl-2-carbonsäure-tert.Butylester und N-Cyclohexyl-formamid/Cyclohexylamin.
Ausbeute: 10 % der Theorie,
$C_{38}H_{44}N_4O_2$ (588,80)
$R_f$-Wert: 0,24 (Kieselgel; Methylenchlorid/Ethanol/Essigsäure = 9:1:0,01)
Massenspektrum: $(M+H)^+$ = 589

Beispiel 34

4'-[[2-n-Propyl-4-methyl-6-[1-(2-dimethylamino-ethyl)-4-isopropyl-5-methyl-imidazol-2-yl]-1H-benzimidazol-1-yl]-methyl]-biphenyl-2-carbonsäure-semihydrat

Hergestellt analog Beispiel 5 aus 4'-[[2-n-Propyl-4-methyl-6-(4-isopropyl-5-methyl-oxazol-2-yl)-1H-benzimidazol-1-yl]-methyl]-biphenyl-2-carbonsäure-tert.butylester und N-(2-Dimethylamino-ethyl)-formamid/2-Dimethylamino-ethylamin.
Ausbeute: 48 % der Theorie,
Schmelzpunkt: 192-195 °C (Zers.)

| $C_{36}H_{43}N_5O_2$ x 0,5 $H_2O$ (586,79) | | | | | | |
|---|---|---|---|---|---|---|
| Ber.:<br>Gef.: | C | 73,69<br>73,53 | H | 7,56<br>7,55 | N | 11,93<br>11,94 |

Massenspektrum: $M^+$ = 577

Beispiel 35

4'-[[2-n-Propyl-4-methyl-6-(1,5-dimethyl-4-isobutyl-imidazol-2-yl)-1H-benzimidazol-1-yl]-methyl]-biphenyl-2-carbonsäure x 0,75 $H_2O$

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-Methyl-formamid/Methylamin.
Ausbeute: 64 % der Theorie,
Schmelzpunkt: 155-157 °C (Zers.)

| $C_{34}H_{38}N_4O_2$ x 0,75 $H_2O$ (548,22) | | | | | | |
|---|---|---|---|---|---|---|
| Ber.:<br>Gef.: | C | 74,49<br>74,45 | H | 7,28<br>7,29 | N | 10,22<br>10,35 |

Massenspektrum: $M^+$ = 534

Beispiel 36

4'-[[2-n-Propyl-4-methyl-6-(1-ethyl-4-isobutyl-5-methyl-imidazol-2-yl)-1H-benzimidazol-1-yl]-methyl]-biphenyl-2-carbonsäure x 0,25 Wasser

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-Ethylformamid/Ethylamin.
Ausbeute: 62 % der Theorie,
Schmelzpunkt: 239-241 °C

| $C_{33}H_{35}N_3O_3$ x 0,25 $H_2O$ (526,17) | | | | | | |
|---|---|---|---|---|---|---|
| Ber. x 0,25 $H_2O$:<br>Gef. : | C | 75,33<br>75,35 | H | 6,80<br>6,75 | N | 7,99<br>7,96 |

Massenspektrum: $M^+$ = 527

Beispiel 37

4'-[[2-n-Propyl-4-methyl-6-(1-tert.butyl-4-isobutyl-5-methylimidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-tert.Butyl-formamid/tert.butylamin.

Beispiel 38

4'-[[2-n-Propyl-4-methyl-6-(1-benzyl-4-isobutyl-5-methyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-Benzylformamid/Benzylamin.

Beispiel 39

4'-[[2-n-Propyl-4-methyl-6-[1-(2-morpholino-ethyl)-4-isobutyl-5-methyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-(2-Morpholino-ethyl)-formamid/2-Morpholino-ethylamin.
Ausbeute: 30 % der Theorie,
Schmelzpunkt: 201-203 °C (Zers.)

| $C_{39}H_{47}N_5O_3$ (633,85) | | | | | | |
|---|---|---|---|---|---|---|
| Ber.:<br>Gef.: | C | 73,90<br>73,65 | H | 7,47<br>7,45 | N | 11,05<br>11,07 |

Massenspektrum: $M^+$ = 633

Beispiel 40

4'-[[2-n-Propyl-4-methyl-6-[1-(2-methoxy-ethyl)-4-isobutyl-5-methyl-imidazol-2-yl]-1H-benzimidazol-1-yl]-methyl]-biphenyl-2-carbonsäure-hydrat

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-(2-Methoxy-ethyl)-formamid/2-Methoxy-ethylamin.
Ausbeute: 29 % der Theorie,
Schmelzpunkt: 135-137 °C (Zers.) (sintert ab 110 °C)

| $C_{36}H_{42}N_4O_3$ x $H_2O$ (596,78) | | | | | | |
|---|---|---|---|---|---|---|
| Ber.:<br>Gef.: | C | 72,46<br>72,50 | H | 7,43<br>7,45 | N | 9,39<br>9,77 |

Massenspektrum: $M^+$ = 578

Beispiel 41

4'-[[2-n-Propyl-4-methyl-6-[1-(2-hydroxy-ethyl)-4-isobutyl-5-methyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-(2-Hydroxy-ethyl)-formamid/2-Hydroxy-ethylamin.

Beispiel 42

4'-[[2-n-Propyl-4-methyl-6-[1-(3-dimethylamino-propyl)-4-isobutyl-5-methyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-(3-Dimethylamino-propyl)-formamid/3-Dimethylamino-propylamin.

Beispiel 43

4'-[[2-n-Propyl-4-methyl-6-(1-carboxymethyl-4-isobutyl-5-methyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-Formylglycinethylester/Glycinethylester.

Beispiel 44

4'-[[2-n-Propyl-4-methyl-6-(1-aminocarbonylmethyl-4-isobutyl-5-methyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und N-Formylglycinamid/Glycinamid.

Beispiel 45

4'-[[2-n-propyl-4-methyl-6-[1-(2-carboxy-ethyl)-4-isobutyl-5-methyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4-isobutyl-5-methyl-oxazol-2-yl)-1H-benzimidazol-1-yl]-methyl]-biphenyl-2-carbonsäure-tert.butylester und 3-Formylamino-propionsäureethylester/3-Aminopropionsäureethylester.

Analog können erhalten werden:
4'-[[2-n-Propyl-4-methyl-6-(1,5-dimethyl-4-isobutyl-imidazol-2-yl)-1H-benzimidazol-1-yl]-methyl]-(1H-tetrazol-5-yl)biphenyl
56-[[2-n-Propyl-4-methyl-6-(1-n-propyl-4-isobutyl-5-methyl-imidazol-2-yl)-1H-benzimidazol-1-yl]-methyl]-(1H-tetrazol-5-yl)biphenyl
4'-[[2-n-Propyl-4-methyl-6-[1-(2-dimethylamino-ethyl)-4-isobutyl-5-methyl-imidazol-2-yl]-1H-benzimidazol-1-yl]-methyl]-(1H-tetrazol-5-yl)biphenyl

Beispiel 46

4'-[[2-n-Propyl-4-methyl-6-(4-methyl-imidazol-2-yl)-1H-benzimidazol-1-yl]-methyl]-biphenyl-2-carbonsaure-methylester

Eine Lösung von 0,35 g (0,7 mMol) 4'-[[2-n-Propyl-4-methyl-6-(4-methyl-imidazolin-2-yl)-1H-benzimidazol-1-yl]-methyl]-biphenyl-2-carbonsäure-methylester in 10 ml Toluol wird unter Stickstoff mit 0,16 g Palladium (10 % auf Aktivkohle) versetzt und 66 Stunden unter Rückfluß erhitzt. Anschließend wird das Toluol abgedampft, der Rückstand in Methylenchlorid aufgenommen, filtriert und eingedampft. Das Rohprodukt wird an Kieselgel (Korngröße: 0,032-0,063 mm) chromatographiert, wobei als Elutionsmittel anfangs Methylenchlorid, später Methylenchlorid/Ethanol/Amoniak (50:1:0,05, 20:1:0,02, 10:1:0,01 und 5:1:0,005) verwendet wird. Die einheitlichen Fraktionen werden vereinigt und eingedampft.
Ausbeute: 0,30 g (9 % der Theorie),
Massenspektrum: $M^+$ = 478

Beispiel 47

4'-[[2-n-Propyl-4-methyl-6-(1,4,5-trimethyl-imidazol-2-yl)-1H-benzimidazol-1-yl]-methyl]-biphenyl-2-carbonsäure x 0,25 $H_2O$

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-Methyl-formamid/Methylamin.
Ausbeute: 61 % der Theorie,
Schmelzpunkt: 217-219 °C (Zers.)

| $C_{31}H_{32}N_4O_2$ x 0,25 $H_2O$ (497,13) | | | | | | |
|---|---|---|---|---|---|---|
| Ber.:<br>Gef.: | C | 74,90<br>74,84 | H | 6,59<br>6,58 | N | 11,27<br>11,26 |

Massenspektrum: $M^+$ = 492

Beispiel 48

4'-[[2-n-Propyl-4-methyl-6-(1-ethyl-4,5-dimethyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-Ethylformamid/Ethylamin.

Beispiel 49

4'-[[2-n-Propyl-4-methyl-6-(1-methyl-4,5-diethyl-imidazol-2-yl)-1H-benzimidazol-1-yl]-methyl]-2-(1H-tetrazol-5-yl)-biphenyl

Hergestellt analog Beispiel 5 aus 4'-[[2-n-Propyl-4-methyl-6-(4,5-diethyl-oxazol-2-yl)-1H-benzimidazol-1-yl]-methyl]-2-(2-triphenylmethyl-tetrazol-5-yl)-biphenyl und N-Methylformamid/Methylamin.

Beispiel 50

4'-[[2-n-Propyl-4-methyl-6-(1-ethyl-4,5-dimethyl-imidazol-2-yl)-1H-benzimidazol-1-yl]-methyl]-2-(1H-tetrazol-5-yl)-biphenyl

Hergestellt analog Beispiel 5 aus 4'-[[2-n-Propyl-4-methyl-6-(4,5-dimethyl-oxazol-2-yl)-1H-benzimidazol-1-yl]-methyl]-2-(2-triphenylmethyl-tetrazol-5-yl)-biphenyl und N-Ethylformamid/Ethylamin.

Beispiel 51

4'-[[2-n-Propyl-4-methyl-6-(1-isopropyl-4,5-dimethyl-imidazol-2-yl)-1H-benzimidazol-1-yl]-methyl]-biphenyl-2-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-Isopropyl-formamid/Isopropylamin.

Beispiel 52

4'-[[2-n-Propyl-4-methyl-6-[1-(2-dimethylamino-ethyl)-4,5-dimethyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-(2-Dimethylamino-ethylformamid/2-Dimeth ylamino-ethylamin.

Beispiel 53

4'-[[2-n-Propyl-4-methyl-6-[1-(2-morpholino-ethyl)-4,5-dimethyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-(2-Morpholino-ethyl)-formamid/2-Morpholino-ethylamin.

Beispiel 54

4'-[[2-n-Propyl-4-methyl-6-[1-(2-morpholino-ethyl)-4,5-diethyl-imidazol-2-yl]-1H-benzimidazol-1-yl]-methyl]-2-(1H-tetrazol-5-yl)-biphenyl

Hergestellt analog Beispiel 5 aus 4'-[[2-n-Propyl-4-methyl-6-(4,5-diethyl-oxazol-2-yl)-1H-benzimidazol-1-yl]-methyl]-2-(2-triphenylmethyl-tetrazol-5-yl)-biphenyl und N-(2-Morpholino-ethyl)-formamid/2-Morpholino-ethylamin.

Beispiel 55

4'-[[2-n-Propyl-4-methyl-6-[1-(2-methoxy-ethyl)-4,5-dimethyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-(2-Methoxy-ethyl)-formamid/2-Methoxy-ethylamin.

Beispiel 56

4'-[[2-n-Propyl-4-methyl-6-[1-(2-methoxy-ethyl)-4,5-dimethyl-imidazol-2-yl]-1H-benzimidazol-1-yl]-methyl]-2-(1H-tetrazol-5-yl)-biphenyl

Hergestellt analog Beispiel 5 aus 4'-[[2-n-Propyl-4-methyl-6-(4,5-dimethyl-oxazol-2-yl)-1H-benzimidazol-1-yl]-methyl]-2-(2-triphenylmethyl-tetrazol-5-yl)-biphenyl und N-(2-Methoxy-ethyl)-formamid/2-Methoxy-ethylamin.

Beispiel 57

4'-[[2-n-Propyl-4-methyl-6-[1-(2-carboxy-ethyl)-4,5-dimethyl-imidazol-2-yl]-1H-benzimidazol-1-yl]-methyl]-2-biphenyl-carbonsäure

Hergestellt analog Beispiel 5 aus 4'-[[2-n-Propyl-4-methyl-6-(4,5-dimethyl-oxazol-2-yl)-1H-benzimidazol-1-yl]-methyl]-biphenyl-2-carbonsäure-tert.butylester und N-Formyl-3-aminopropionsäure-ethylester/3-Aminopropionsäure-ethylester.

Beispiel 58

4'-[[2-n-Propyl-4-methyl-6-(1-methyl-4,5-diethyl-imidazol-2-yl)-1H-benzimidazol-1-yl]-methyl]-biphenyl-2-carbonsäure x 0,25 $H_2O$

Hergestellt analog Beispiel 5 aus 4'-[[2-n-Propyl-4-methyl-6-(4,5-diethyl-oxazol-2-yl)-1H-benzimidazol-1-yl]-methyl]-biphenyl-2-carbonsäure-tert.butylester und N-Methyl-formamid/Methylamin.
Ausbeute: 65 % der Theorie,
Schmelzpunkt: 247-249 °C (Zers.)

| $C_{33}H_{36}N_4O_2$ x 0,25 $H_2O$ (525,18) | | | | | | |
|---|---|---|---|---|---|---|
| Ber.:<br>Gef.: | C | 75,47<br>75,43 | H | 7,01<br>7,11 | N | 10,67<br>10,68 |

Massenspektrum: $M^+$ = 520

Beispiel 59

4'-[[2-n-Propyl-4-methyl-6-(1-methyl-imidazolin-2-yl)-1H-benzimidazol-1-yl]-methyl]-biphenyl-2-carbonsäure

Hergestellt durch Erhitzen von 4'-[[2-n-Propyl-4-methyl-6-[2-(N-methylamino)-ethylaminocarbonyl]-1H-benzimidazol-1-yl]methyl]-biphenyl-2-carbonsäure-tert.butylester in Phosphoroxychlorid und Isolierung analog Beispiel 10. Ausbeute: 30 % der Theorie,
$C_{29}H_{30}N_4O_2$ (466,59)
$R_f$-Wert: 0,50 (Methylenchlorid/Methanol/Essigsäure = 2:1:0,02)
Massenspektrum: $(M+H)^+$ = 467

Beispiel 60

4'-[[2-n-Propyl-4-methyl-6-[1-(2-dimethylamino-ethyl)-4,5-diethyl-imidazol-2-yl]-1H-benzimidazol-1-yl]-methyl]-biphenyl-2-carbonsäure-dihydrat-trihydrochlorid

Hergestellt analog Beispiel 5 aus 4'-[[2-n-Propyl-4-methyl-6-(4,5-diethyl-oxazol-2-yl)-1H-benzimidazol-1-yl]-methyl]-biphenyl-2-carbonsäure-tert.butylester und N-(2-Dimethylamino-ethyl)-formamid/2-Dimethylamino-ethylamin.
Ausbeute: 14 % der Theorie,
Schmelzpunkt: ab 210 °C (Zers.)

| $C_{36}H_{43}N_5O_2$ x 3 HCl x 2 $H_2O$ (723,21) | | | | | | | | |
|---|---|---|---|---|---|---|---|---|
| Ber.:<br>Gef.: | C | 59,79<br>59,28 | H | 6,97<br>6,92 | N | 9,69<br>9,75 | Cl | 14,71<br>14,26 |

Massenspektrum: $M^+$ = 577

Beispiel 61

4'-[[2-n-Propyl-4-methyl-6-[1-(2-morpholino-ethyl)-4,5-diethyl-imidazol-2-yl]-1H-benzimidazol-1-yl]-methyl]-biphenyl-2-carbonsäure

Hergestellt analog Beispiel 5 aus 4'-[[2-n-Propyl-4-methyl-6-(4,5-diethyl-oxazol-2-yl)-1H-benzimidazol-1-yl]-methyl]-biphenyl-2-carbonsäure-tert.butylester und N-(2-Morpholino-ethyl)-formamid/2-Morpholino-ethylamin.
Ausbeute: 41 % der Theorie,
Schmelzpunkt: ab 196 °C (Zers.)
$C_{38}H_{45}N_5O_3$ (619,82)
Massenspektrum: $M^+$ = 619

Beispiel 62

4'-[[2-n-Propyl-4-methyl-6-[1-(2-methoxy-ethyl)-4,5-diethyl-imidazol-2-yl]-1H-benzimidazol-1-yl]-methyl]-biphenyl-2-carbonsäure-hydrat

Hergestellt analog Beispiel 5 aus 4'-[[2-n-Propyl-4-methyl-6-(4,5-diethyl-oxazol-2-yl)-1H-benzimidazol-1-yl]-methyl]-biphenyl-2-carbonsäure-tert.butylester und N-(2-Methoxy-ethyl)-formamid/2-Methoxy-ethylamin.
Ausbeute: 46 % der Theorie,
Schmelzpunkt: ab 132 °C (Zers.)

| $C_{35}H_{40}N_4O_3$ x $H_2O$ (582,76) | | | | | | |
|---|---|---|---|---|---|---|
| Ber. x $H_2O$:<br>Gef.: | C | 72,14<br>71,99 | H | 7,27<br>7,19 | N | 9,61<br>9,84 |

Massenspektrum: $(M-H)^-$ = 563

Beispiel 63

4'-[[2-n-Propyl-4-methyl-6-(4,5-diethyl-oxazol-2-yl)-1H-benzimidazol-1-yl]-methyl]-biphenyl-2-carbonsäure

Hergestellt analog Beispiel 29 aus 4'-[[2-n-Propyl-4-methyl-6-[N-(1-propionyl-n-propyl)-aminocarbonyl]-1H-benzimidazol-1-yl]-methyl]-biphenyl-2-carbonsäure-tert.butylester und Phosphoroxychlorid.
Ausbeute: 97 % der Theorie,
Schmelzpunkt: 250-255 ° C

| $C_{32}H_{33}N_3O_3$ (507,64) | | | | | | |
|---|---|---|---|---|---|---|
| Ber.:<br>Gef.: | C | 75,71<br>75,77 | H | 6,55<br>6,59 | N | 8,28<br>8,46 |

Massenspektrum: $M^+$ = 507

Beispiel 64

4'-[[2-n-Propyl-4-methyl-6-(4,5-diethyl-1H-imidazol-2-yl)-1H-benzimidazol-1-yl]-methyl]-2-(1H-tetrazol-5-yl)-biphenyl

Hergestellt analog Beispiel 25b aus 4'-[[2-n-Propyl-4-methyl-6-[N-(1-propionyl-n-propyl)-aminocarbonyl]-1H-benzimidazol-1-yl]-methyl]-2-(2-triphenylmethyl-tetrazol-5-yl)-biphenyl und Ammoniumacetat/Eisessig.
Ausbeute: 27 % der Theorie,
Schmelzpunkt: 221-223 ° C

| $C_{32}H_{34}N_8$ x $H_2O$ (530,69) | | | | | | |
|---|---|---|---|---|---|---|
| Ber.:<br>Gef.: | C | 70,05<br>70,79 | H | 6,61<br>6,98 | N | 20,42<br>18,83 |

Massenspektrum: $M^+$ = 530

Beispiel 65

4'-[[2-n-Propyl-4-methyl-6-(4,5-dimethyl-oxazol-2-yl)-1H-benzimidazol-1-yl]-methyl]-biphenyl-2-carbonsäure

Hergestellt analog Beispiel 29 aus 4'-[[2-n-Propyl-4-methyl-6-[N-(1-acetyl-ethyl)-aminocarbonyl]-1H-benzimidazol-1-yl]-methyl]-biphenyl-2-carbonsäure-tert.butylester und Phosphoroxychlorid.
Ausbeute: 88 % der Theorie,
Schmelzpunkt: 259-260 ° C (Zers.)

| $C_{30}H_{29}N_3O_3$ x 0,25 $H_2O$ (484,09) | | | | | | |
|---|---|---|---|---|---|---|
| Ber.:<br>Gef.: | C | 74,44<br>74,33 | H | 6,14<br>6,14 | N | 8,68<br>8,69 |

Massenspektrum: $M^+$ = 479

Beispiel 66

4'-[[2-n-Propyl-4-methyl-6-(1-methyl-5,6,7,8-tetrahydrobenzimidazol-2-yl)-1H-benzimidazol-1-yl]-methyl]-2'-(2,5-dihydro-5-oxo-1,2,4-oxadiazol-3-yl)-biphenyl

a) 4'-[[2-n-Propyl-4-methyl-6-(1-methyl-5,6,7,8-tetrahydrobenzimidazol-2-yl)-1H-benzimidazol-1-yl]-methyl]-2'-(hydroxycarbamimidoyl)-biphenyl

Zu einer Lösung von 8,4 g (0,12 Mol) Hydroxylamin-hydrochlorid in 30 ml Dimethylsulfoxid werden bei Raumtemperatur 30 ml einer 30%igen Lösung von Natriummethanolat in Methanol zugetropft. Nach weiteren 10 Minuten werden 4,5 g (10 mMol) 4'-[[2-n-Propyl-4-methyl-6-(1-methyl-5,6,7,8-tetrahydrobenzimidazol-2-yl)-1H-benzimidazol-1-yl]-methyl]-2'-cyano-biphenyl zugegeben, die dabei entstandene Suspension wird 12 Stunden auf 90°C erhitzt. Nach Abkühlung auf Raumtemperatur wird die Reaktionsmischung auf 200 ml Eiswasser gegossen, der dabei gebildete Niederschlag wird abgesaugt, mit Wasser gewaschen, in Methylenchlorid aufgenommen und an Kieselgel (Korngröße: 0,023-0,063 mm) chromatographiert, wobei als Elutionsmittel Gemische von Essigester, Ethanol und konz. Ammoniak (19:1:0,06, 19:1:0,08, 19:1:0,1 und 9:1:0,2) verwendet werden. Die einheitlichen Fraktionen werden vereinigt, eingedampft, mit Ether verrieben und getrocknet.
Ausbeute: 1,2 g (25 % der Theorie),
Schmelzpunkt: 221-224°C (Zers.)

b) 4'-[[2-n-Propyl-4-methyl-6-(1-methyl-5,6,7,8-tetrahydrobenzimidazol-2-yl)-1H-benzimidazol-1-yl]-methyl]-2'-(2,5-dihydro-5-oxo-1,2,4-oxadiazol-3-yl)-biphenyl

Zu einer Lösung von 0,5 g (1 mMol) 4'-[[2-n-Propyl-4-methyl-6-(1-methyl-5,6,7,8-tetrahydrobenzimidazol-2-yl)-1H-benzimidazol-1-yl]-methyl]-2'-(hydroxycarbamimidoyl)-biphenyl und 0,14 ml (1 mMol) Triethylamin in 40 ml Tetrahydrofuran wird bei 5°C eine Lösung von 0,1 ml (1 mMol) Chlorameisensäureethylester in 1 ml Methylenchlorid zugetropft. Nach 2 Stunden bei Raumtemperatur wird vom gebildeten Niederschlag abgesaugt. Das Filtrat wird eingedampft, der erhaltene Rückstand wird in 5 ml Xylol aufgenommen und 90 Minuten unter Rückfluß erhitzt. Nach Abkühlung auf Raumtemperatur wird das Reaktionsgemisch mit 20 ml Essigester versetzt, mit Wasser gewaschen und über Magnesiumsulfat getrocknet. Das Rohprodukt wird an Kieselgel (Korngröße: 0,032-0,063 mm) chromatographiert, wobei als Elutionsmittel Methylenchlorid mit steigenden Mengen Ethanol (0 bis 10 %) verwendet werden. Die einheitlichen Fraktionen werden vereinigt, eingedampft, mit Ether verrieben und getrocknet.
Ausbeute: 80 mg (14 % der Theorie),
Schmelzpunkt: ab 199°C (Zers.)
$R_f$-Wert: 0,33 (Kieselgel; Essigester/Methanol = 3:1)
$C_{34}H_{34}N_6O_2$ (558,69)
Massenspektrum: $(M-H)^-$ = 557

Analog Beispiel 66 wird folgende Verbindung erhalten:
4'-[[2-n-Propyl-4--methyl-6-(1,4,5-trimethyl-imidazol-2-yl)-1H-benzimidazol-1-yl]-methyl]-2'-(2,5-dihydro-5-oxo-1,2,4-oxadiazol-3-yl)-biphenyl

Bei den nachfolgenden pharmazeutischen Anwendungsbeispielen kann als Wirksubstanz jede geeignete Verbindung der Formel I, insbesondere diejenigen in denen $R_5$ eine in vivo in eine Carboxygruppe metabolisierbare Gruppe, eine Carboxy- oder 1H-Tetrazolylgruppe darstellt, eingesetzt werden:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 °C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40 °C wird die gemahlene Wirksubstanz in der Schmelze homogen dispergiert. Es wird auf 38 °C abgekühlt und in schwach vorgekühlte Suppositorienformen ausgegossen.

**Patentansprüche**

**1.** Benzimidazole der allgemeinen Formel

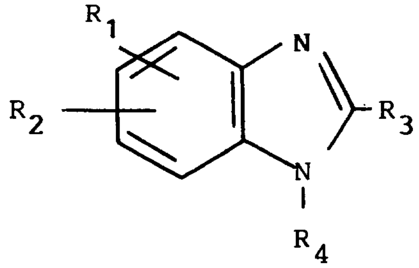

,(I)

in der
$R_1$ ein Wasserstoff-, Fluor-, Chlor- oder Bromatom, eine Fluormethyl-, Difluormethyl-, Trifluormethyl- oder Alkylgruppe,
$R_2$ eine gegebenenfalls in 1-Stellung durch den Rest $R_a$ substituierte Imidazol-2-yl-Gruppe, wobei
$R_a$ eine Phenyl- oder Phenylalkylgruppe, in der der Phenylkern jeweils durch Alkyl-, Hydroxy- oder Alkoxygruppen mono- oder disubstituiert sein kann und die Substituenten gleich oder verschieden sein

können, eine Cycloalkylgruppe mit 3 bis 7 Kohlenstoffatomen oder eine Alkylgruppe mit 1 bis 6 Kohlenstoffatomen, in der der Alkylteil zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe oder ab Position 2 durch eine Hydroxy-, Alkoxy-, Amino-, Alkylamino-, Dialkylamino-, Pyrrolidino-, Piperidino- oder Morpholinogruppe substituiert sein kann, darstellt,

eine 5,5-Spiro-cyclopentano-dihydro-imidazol-4-on-2-yl-gruppe,

eine in 1- und 3-Stellung durch den Rest $R_b$, der gleich oder verschieden sein kann, substituierte Imidazolium-2-yl-Gruppe, wobei

$R_b$ eine Phenylalkylgruppe, in der der Phenylkern durch Alkyl-, Hydroxy- oder Alkoxygruppen mono- oder disubstituiert sein kann und die Substituenten gleich oder verschieden sein können, oder eine Alkylgruppe mit 1 bis 6 Kohlenstoffatomen, in der der Alkylteil zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, darstellt,

eine Oxazol-2-yl- oder Thiazol-2-yl-Gruppe, wobei in den vorstehend erwähnten Imidazol-2-yl-, Imidazolium-2-yl-, Oxazol-2-yl- oder Thiazol-2-yl-teilen jeweils die 4-, 5-Positionen durch eine Alkylgruppe mit 1 bis 5 Kohlenstoffatomen oder durch eine Phenylgruppe substituiert sein können, wobei die Substituenten gleich oder verschieden sein können, oder auch über die 4,5-Positionen eine n-Propylen- oder n-Butylenbrücke angefügt sein kann,

eine in 4-Stellung durch $R_9$ und $R_{10}$ und in 5-Stellung durch $R_{10}$ substituierte Oxazolin-2-yl- oder Imidazolin-2-yl-Gruppe,wobei eine Iminogruppe zusätzlich durch $R_a$ substituiert sein kann, oder eine $R_8CO-(R_9CR_{10})-NR_a-CO$-Gruppe, in denen

$R_a$ wie vorstehend erwähnt definiert ist,

$R_8$ bis $R_{10}$, die gleich oder verschieden sein können, Wasserstoffatome, Alkylgruppen mit 1 bis 5 Kohlenstoffatomen oder Phenylgruppen darstellen,

$R_3$ eine Alkylgruppe mit 1 bis 5 Kohlenstoffatomen, eine Cycloalkylgruppe mit 3 bis 5 Kohlenstoffatomen, eine Alkoxy- oder Alkylthiogruppe mit jeweils 1 bis 4 Kohlenstoffatomen und

$R_4$ ein Wasserstoffatom oder eine Gruppe der allgemeinen Formel

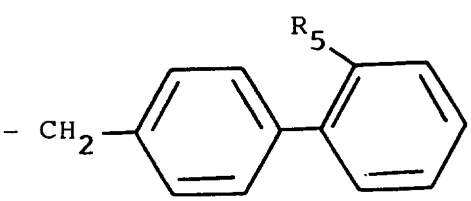

,

in welcher

$R_5$ eine in vivo in eine Carboxygruppe metabolisierbare Gruppe, eine Carboxy-, Cyano-, 2,5-Dihydro-5-oxo-1,2,4-oxadiazol-3-yl-, 1H-Tetrazolyl-, 1-Triphenylmethyl-tetrazolyl- oder 2-Triphenylmethyl-tetrazolylgruppe darstellt,

bedeuten, wobei alle vorstehend erwähnten Alkyl- und Alkoxygruppen, soweit nichts anderes erwähnt wurde, jeweils 1 bis 3 Kohlenstoffatome enthalten können und

unter dem vorstehend erwähnten Begriff "eine in-vivo in eine Carboxygruppe metabolisierbare Gruppe" deren Ester der Formeln

- CO - OR',
- CO - O - (HCR'') - O - CO - R''' und
- CO - O - (HCR'') - O - CO - OR'''

in denen

R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,

R'' ein Wasserstoffatom oder eine Methylgruppe und

R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe darstellen, zu verstehen sind, deren 3-Isomere, deren 1-, 3-Isomerengemische und deren Salze.

**2.** Benzimidazole der allgemeinen Formel I gemäß Anspruch 1, in der
$R_1$ ein Wasserstoff-, Fluor-, Chlor- oder Bromatom, eine Trifluormethylgruppe oder eine Alkylgruppe mit 1 bis 3 Kohlenstoffatomen,
$R_2$ eine gegebenenfalls in 1-Stellung durch den Rest $R_a$ substituierte zmidazol-2-yl-Gruppe, wobei
$R_a$ eine Phenylgruppe, eine Phenylalkylgruppe mit 1 bis 3 Kohlenstoffatomen im Alkylteil, eine Cycloalkylgruppe mit 5 bis 7 Kohlenstoffatomen oder eine Alkylgruppe mit 1 bis 5 Kohlenstoffatomen, in der der Alkylteil zusätzlich durch eine in vivo in eine Carboxygruppe metabolisierbare Gruppe, durch eine Trifluormethyl-, Carboxyl-, Methoxycarbonyl-, Ethoxycarbonyl-, Aminocarbonyl-, Methylaminocarbonyl- oder Dimethylaminocarbonylgruppe oder ab Position 2 durch eine Hydroxy-, Methoxy-, Ethoxy-, Amino-, Methylamino-, Dimethylamino-, Pyrrolidino-, Piperidino- oder Morpholinogruppe substituiert sein kann, darstellt,
eine 5,5-Spiro-cyclopentano-dihydro-imidazol-4-on-2-yl-gruppe,
eine in 1- und 3-Stellung durch den Rest $R_b$, der gleich oder verschieden sein kann, substituierte Imidazolium-2-yl-Gruppe, wobei
$R_b$ eine Alkyl- oder Phenylalkylgruppe mit jeweils 1 bis 3 Kohlenstoffatomen im Alkylteil darstellt,
eine Oxazol-2-yl- oder Thiazol-2-yl-Gruppe, wobei in den vorstehend erwähnten Imidazol-2-yl-, Imidazolium-2-yl-, Oxazol-2-yl- oder Thiazol-2-yl-teilen jeweils die 4-, 5-Positionen durch eine Alkylgruppe mit 1 bis 4 Kohlenstoffatomen oder durch eine Phenylgruppe substituiert sein können, wobei die Substituenten gleich oder verschieden sein können, oder auch über die 4,5-Positionen eine n-Butylenbrücke angefügt sein kann,
eine durch $R_9$ bis $R_{10}$ substituierte Oxazolin-2-yl- oder Imidazolin-2-yl-Gruppe, wobei zusätzlich eine Iminogruppe durch $R_a$ substituiert sein kann und $R_9$ bis $R_{10}$ und $R_a$ jeweils ein Wasserstoffatom oder eine Alkylgruppe mit 1 bis 4 Kohlenstoffatomen darstellen,
$R_3$ eine Alkylgruppe mit 2 bis 5 Kohlenstoffatomen, eine Cycloalkylgruppe mit 3 bis 5 Kohlenstoffatomen, eine Alkoxy- oder Alkylthiogruppe mit jeweils 2 bis 4 Kohlenstoffatomen und
$R_4$ eine Gruppe der allgemeinen Formel

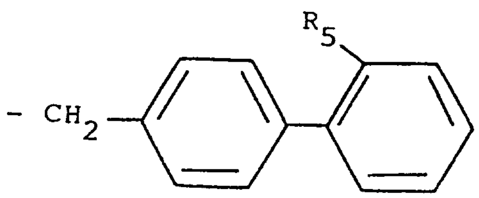 ,

in welcher
$R_5$ eine in vivo in eine Carboxygruppe metabolisierbare Gruppe, eine Carboxy-, Cyano-, 2,5-Dihydro-5-oxo-1,2,4-oxadiazol-3-yl-, 1H-Tetrazolyl, 1-Triphenylmethyl-tetrazolyl- oder 2-Triphenylmethyl-tetrazolylgruppe darstellt,
bedeuten, wobei unter dem vorstehend erwähnten Begriff "eine in-vivo in eine Carboxygruppe metabolisierbare Gruppe" deren Ester der Formeln

- CO - OR',
- CO - O - (HCR'') - O - CO - R''' und
- CO - O - (HCR'') - O - CO - OR'''

in denen
R' eine geradkettige oder verzweigte Alkylgruppe mit 1 bis 4 Kohlenstoffatomen, eine Cycloalkylgruppe mit 5 bis 7 Kohlenstoffatomen,
R'' ein Wasserstoffatom oder eine Methylgruppe und
R''' eine geradkettige oder verzweigte Alkylgruppe mit 1 bis 4 Kohlenstoffatomen oder eine Cycloalkylgruppe mit 5 bis 7 Kohlenstoffatomen bedeuten, zu verstehen sind,
deren 3-Isomere, deren 1-, 3-Isomerengemische und deren Salze.

**3.** Benzimidazole der allgemeinen Formel I gemäß Anspruch 1, in der
$R_3$ und $R_4$ wie im Anspruch 2 definiert sind,
$R_2$ in 6-Stellung die im Anspruch 2 erwähnten Bedeutungen aufweist und
$R_1$ in 4-Stellung ein Fluor-, Chlor- oder Bromatom, eine Trifluormethylgruppe oder eine Alkylgruppe mit 1 bis 3 Kohlenstoffatomen bedeuten,
und deren Salze.

**4.** Benzimidazole der allgemeinen Formel I gemäß Anspruch 1, in der
$R_1$ bis $R_4$ wie im Anspruch 3 definiert sind und
$R_2$ in 6-Stellung einen der im Anspruch 3 erwähnten Imidazolylreste darstellt,
und deren Salze.

**5.** Folgende Verbindungen der allgemeinen Formel I gemäß Anspruch 1:
(a) 4'-[[2-n-Propyl-4-methyl-6-(5,6,7,8-tetrahydrobenzimidazol-2-yl)-1H-benzimidazol-1-yl]-methyl]-biphenyl-2-carbonsäure,
(b) 4'-[[2-n-Propyl-4-methyl-6-(1-methyl-5,6,7,8-tetrahydrobenzimidazol-2-yl)-1H-benzimidazol-1-yl]-methyl]-biphenyl-2-carbonsäure,
(c) 4'-[[2-n-Propyl-4-methyl-6-(1-methyl-5,6,7,8-tetrahydrobenzimidazol-2-yl)-1H-benzimidazol-1-yl]-methyl]-2-(1H-tetrazol-5-yl)biphenyl,
(d) 4'-[[2-n-Propyl-4-methyl-6-(1-benzyl-5,6,7,8-tetrahydrobenzimidazol-2-yl)-1H-benzimidazol-1-yl]-methyl]-biphenyl-2-carbonsäure,
(e) 4'-[[2-Ethyl-4-methyl-6-(1-phenyl-5,6,7,8-tetrahydrobenzimidazol-2-yl)-1H-benzimidazol-1-yl]-methyl]-biphenyl-2-carbonsäure,
(f) 4'-[[2-n-Propyl-4-methyl-6-(1-carboxymethyl-5,6,7,8-tetrahydrobenzimidazol-2-yl)-1H-benzimidazol-1-yl]-methyl]-biphenyl-2-carbonsäure,
(g) 4'-[[2-Ethyl-4-methyl-6-(1-ethyl-5,6,7,8-tetrahydro-benzimidazol-2-yl)-1H-benzimidazol-1-yl]-methyl]-2-(1H-tetrazol-5-yl)-biphenyl,
(h) 4'-[[2-n-Propyl-4-methyl-6-(4,4-dimethyl-oxazolin-2-yl)-1H-benzimidazol-1-yl]-methyl]-biphenyl-2-carbonsäure,
(i) 4'-[[2-n-Propyl-4-methyl-6-(1,5-dimethyl-4-phenyl-imidazol-2-yl)-1H-benzimidazol-1-yl]-methyl]-biphenyl-2-carbonsäure und
(k) 4'-[[2-n-Propyl-4-methyl-6-(4-isopropyl-1,5-dimethyl-imidazol-2-yl)-1H-benzimidazol-1-yl]-methyl]-biphenyl-2-carbonsäure
sowie deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels mit Angiotensin-antagonistischer Wirkung.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Benzimidazole der allgemeinen Formel I gemäß den Ansprüchen 1 bis 6, dadurch gekennzeichnet, daß
a) zur Herstellung von Verbindungen der allgemeinen Formel I, in der $R_2$ eine Oxazol-2-yl-, Thiazol-2-yl- oder Imidazol-2-yl-Gruppe darstellt, in denen jeweils über die 4,5-Positionen eine n-Butylenbrücke angefügt ist und zusätzlich die Iminogruppe im Imidazolring durch eine Alkylgruppe mit 1 bis 6 Kohlenstoffatomen, durch eine Phenylalkylgruppe mit 1 bis 3 Kohlenstoffatomen im Alkylteil oder durch eine Phenylgruppe substituiert sein kann, eine Verbindung der allgemeinen Formel

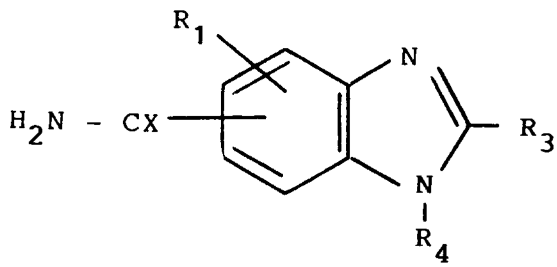

,(II)

in der
$R_1$, $R_3$ und $R_4$ wie in den Ansprüchen 1 bis 5 definiert sind und
X ein Sauerstoff- oder Schwefelatom oder eine gegebenenfalls durch eine Alkylgruppe mit 1 bis 6 Kohlenstoffatomen, durch eine Phenylalkylgruppe mit 1 bis 3 Kohlenstoffatomen im Alkylteil oder durch eine Phenylgruppe substituierte Iminogruppe darstellt, mit einem α-Halogenketon der allgemeinen Formel

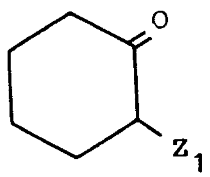

, (III)

in der
$Z_1$ ein Halogenatom darstellt, umgesetzt wird oder
b) zur Herstellung von Verbindungen der allgemeinen Formel I, in der $R_2$ eine der in den Ansprüchen 1 bis 5 erwähnten Imidazol-2-yl-Gruppen darstellt, die in 1-Stellung durch den Rest $R_a$ substituiert sein können, eine Verbindung der allgemeinen Formel

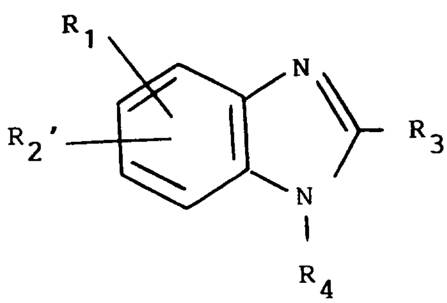

, (IV)

in der
$R_1$, $R_3$ und $R_4$ wie in den Ansprüchen 1 bis 5 definiert sind und
$R_2'$ eine der in den Ansprüchen 1 bis 5 erwähnten Oxazol-2-yl-Gruppen darstellt, mit einem Amin der allgemeinen Formel

$H_2N - R_6$ , (V)

in der
$R_6$ die für $R_a$ in den Ansprüchen 1 bis 5 erwähnten Bedeutungen besitzt oder ein Wasserstoffatom darstellt, umgesetzt wird oder
c) zur Herstellung von Benzimidazolen der allgemeinen Formel I, in der $R_4$ eine Gruppe der Formel

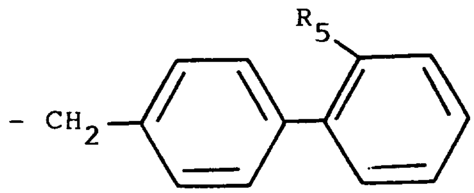

darstellt,
ein Benzimidazol der allgemeinen Formel

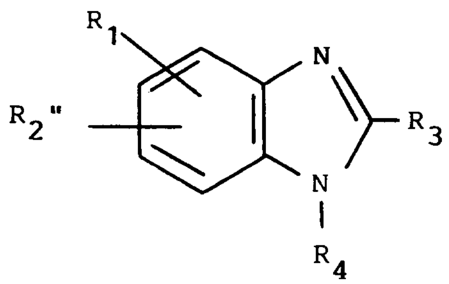

, (VI)

in der
$R_1$, $R_3$ und $R_4$ wie in den Ansprüchen 1 bis 5 definiert sind und
$R_2$" mit Ausnahme der in 1-Stellung unsubstituierten Imidazol-2-yl- und Imidazolin-2-yl-Gruppe die für $R_2$ in den Ansprüchen 1 bis 5 erwähnten Bedeutungen besitzt, mit einer Biphenylverbindung der allgemeinen Formel

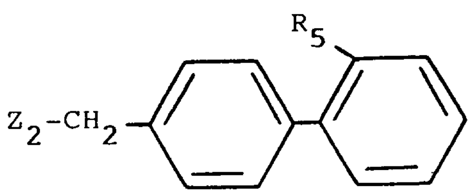

, (VII)

in der
$R_5$ wie in den Ansprüchen 1 bis 5 definiert ist und
$Z_2$ eine nukleophile Austrittsgruppe darstellt, umgesetzt wird oder
d) zur Herstellung einer Verbindung der allgemeinen Formel I, in der $R_5$ eine Carboxygruppe darstellt, eine Verbindung der allgemeinen Formel

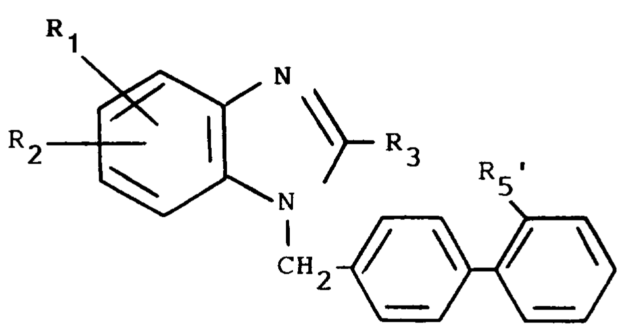

, (VIII)

in der
$R_1$ bis $R_3$ wie in den Ansprüchen 1 bis 5 definiert sind und
$R_5$' eine mittels Hydrolyse, Thermolyse oder Hydrogenolyse in eine Carboxygruppe überführbare Gruppe darstellt, in eine Verbindung der allgemeinen Formel I, in der $R_5$ eine Carboxygruppe

darstellt, übergeführt wird oder
e) zur Herstellung von Verbindungen der allgemeinen Formel I, in der $R_2$ eine 5,5-Spiro-cyclopentano-dihydro-imidazol-4-on-2-yl-Gruppe darstellt, ein Benzimidazol der allgemeinen Formel

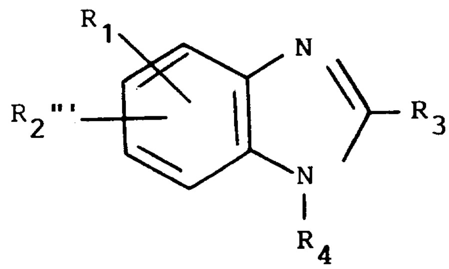 , (IX)

in der
$R_1$, $R_3$ und $R_4$ wie in den Ansprüchen 1 bis 5 definiert sind und
$R_2$''' eine Imidazol-2-yl-Gruppe darstellt, in der über die 4,5-Positionen eine n-Butylenbrücke angefügt ist, mit einer Base in Gegenwart von Luft und Licht behandelt wird oder
f) zur Herstellung einer Verbindung der allgemeinen Formel I, in der $R_5$ eine 1H-Tetrazolylgruppe darstellt, ein Schutzrest von einer Verbindung der allgemeinen Formel

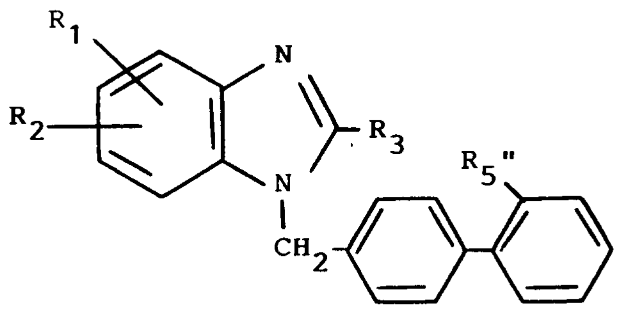 , (X)

in der
$R_1$, $R_2$ und $R_3$ wie in den Ansprüchen 1 bis 5 definiert sind und
$R_5$'' eine in 1- oder 2-Stellung durch einen Schutzrest geschützte 1H-Tetrazolyl- oder 2H-Tetrazolylgruppe darstellt, abgespalten wird oder
g) zur Herstellung einer Verbindung der allgemeinen Formel I, in der $R_5$ eine 1H-Tetrazolylgruppe darstellt, eine Verbindung der allgemeinen Formel

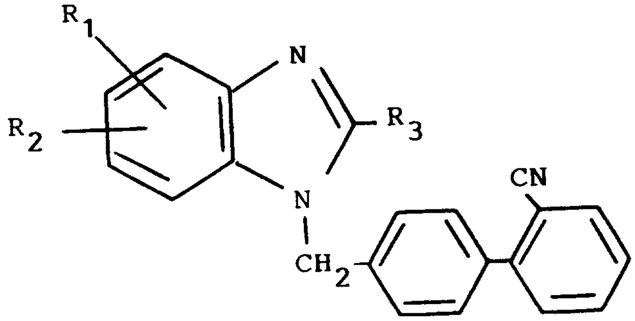 , (XI)

in der
$R_1$ bis $R_3$ wie in den Ansprüchen 1 bis 5 definiert sind, mit Stickstoffwasserstoffsäure oder deren Salzen umgesetzt wird oder
h) zur Herstellung von Verbindungen der allgemeinen Formel I, in der $R_2$ eine der in den Ansprüchen 1 bis 5 erwähnten Imidazol-2-yl-Gruppen darstellt, die in 1-Stellung durch eine Phenylalkylgruppe, wobei der Phenylkern durch Alkyl-, Hydroxy- oder Alkoxygruppen mono- oder disubstituiert sein kann und die Substituenten gleich oder verschieden sein können, oder durch eine Alkylgruppe mit 1 bis 6 Kohlenstoffatomen, wobei die Alkylgruppe zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, oder eine der in den Ansprüchen 1 bis 5 erwähnten Imidazolium-2-yl-Gruppen darstellt, eine Verbindung der allgemeinen Formel

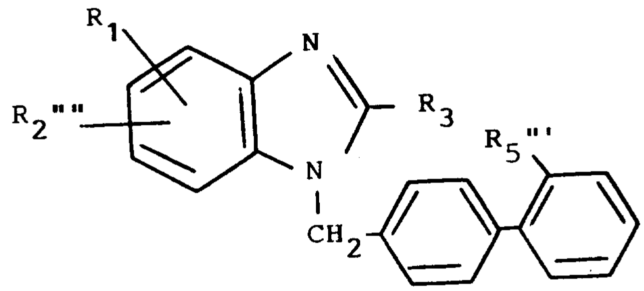

, (XII)

in der
$R_1$ und $R_3$ wie in den Ansprüchen 1 bis 5 definiert sind,
$R_2''''$ eine der in 1-Stellung in den Ansprüchen 1 bis 5 erwähnten unsubstituierten Imidazol-2-yl-Gruppen und
$R_5'''$ eine Carboxygruppe oder eine mittels Hydrolyse, Thermolyse oder Hydrogenolyse in eine Carboxygruppe überführbare Gruppe oder eine durch einen Schutzrest geschützte 1H-Tetra-zolyl- oder 2H-Tetrazolylgruppe darstellen, mit einer Verbindung der allgemeinen Formel

$Z_3$ - $R_7$ , (XIII)

in der
$R_7$ eine Phenylalkylgruppe, wobei der Phenylkern durch Alkyl-, Hydroxy- oder Alkoxygruppen mono- oder disubstituiert sein kann und die Substituenten gleich oder verschieden sein können, oder eine Alkylgruppe mit 1 bis 6 Kohlenstoffatomen, wobei die Alkylgruppe zusätzlich durch eine in vivo in eine Carboxygruppe metabolisierbare Gruppe, durch eine Trifluormethyl-, Carboxyl-, Alkoxycarbonyl-, Aminocarbonyl-, Alkylaminocarbonyl- oder Dialkylaminocarbonylgruppe substituiert sein kann, und
$Z_3$ eine nukleophile Austrittsgruppe bedeuten, umgesetzt und erforderlichenfalls anschließend ein verwendeter Schutzrest abgespalten wird oder
i) zur Herstellung von Verbindungen der allgemeinen Formel I, in der $R_2$ einen der durch die Reste $R_8$ bis $R_{10}$ substituierten in den Ansprüchen 1 bis 5 erwähnten Imidazol-2-yl-Reste darstellt, wobei jedoch $R_9$ oder $R_{10}$ ein Wasserstoffatom darstellen muß, ein Aminoketon der allgemeinen Formel

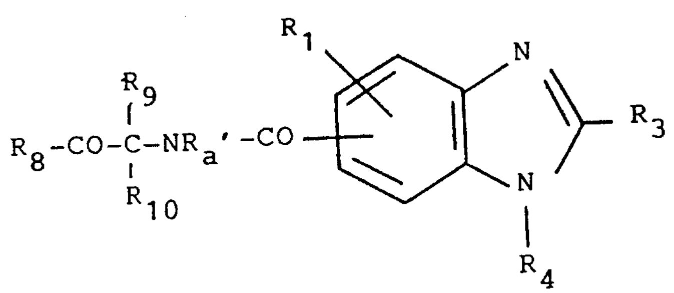

,(XIV)

in der
$R_1$, $R_3$, $R_4$ und $R_8$ bis $R_{10}$ wie in den Ansprüchen 1 bis 5 definiert sind, wobei jedoch $R_9$ oder $R_{10}$ ein Wasserstoffatom darstellen muß, und
$R_a'$ die für $R_a$ in den Ansprüchen 1 bis 5 erwähnten Bedeutungen besitzt und ein Wasserstoffatom darstellt, mit einem Ammoniumsalz einer niederen aliphatischen Carbonsäure umgesetzt wird oder
j) zur Herstellung von Verbindungen der allgemeinen Formel I, in der $R_2$ einen der in den Ansprüchen 1 bis 5 erwähnten Oxazolin-2-yl- oder Imidazolin-Reste darstellt, eine Verbindung der

allgemeinen Formel

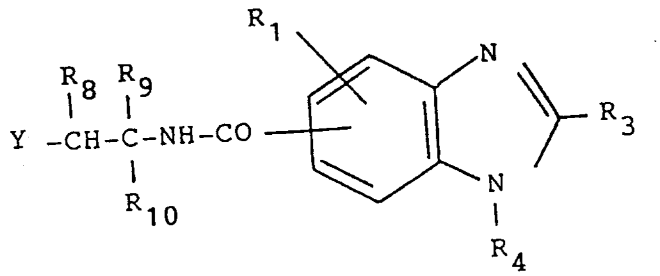

,(XV)

in der
$R_1$, $R_3$, $R_4$ und $R_8$ bis $R_{10}$ wie in den Ansprüchen 1 bis 5 definiert sind und
Y eine Hydroxy- oder $HNR_a$-Gruppe darstellt, wobei $R_a$ wie in den Ansprüchen 1 bis 5 definiert ist, dehydratisiert wird oder

k) zur Herstellung von Verbindungen der allgemeinen Formel I, in der $R_2$ einen der in den Ansprüchen 1 bis 5 erwähnten Imidazolin-2-yl-Reste darstellt, eine Verbindung der allgemeinen Formel

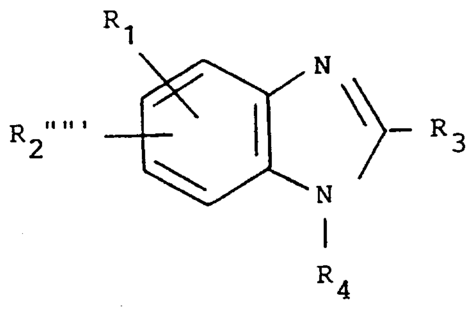

,(XVI)

in der
$R_1$, $R_3$ und $R_4$ wie in den Ansprüchen 1 bis 5 definiert sind und $R_2$''''' einen der in den Ansprüchen 1 bis 5 erwähnten Oxazolin-2-yl-Reste darstellt, mit einem Amin der allgemeinen Formel

$H_2N\text{-}(R_8CH)\text{-}(R_9CR_{10})\text{-}NH_2$ ,(XVII)

in der
$R_8$ bis $R_{10}$ wie in den Ansprüchen 1 bis 5 definiert sind, umgesetzt wird oder

l) zur Herstellung von Verbindungen der allgemeinen Formel I, in der $R_2$ einen der durch die Reste $R_8$ bis $R_{10}$ substituierten in den Ansprüchen 1 bis 5 erwähnten Oxazol-2-yl-Reste darstellt, ein Aminoketon der allgemeinen Formel

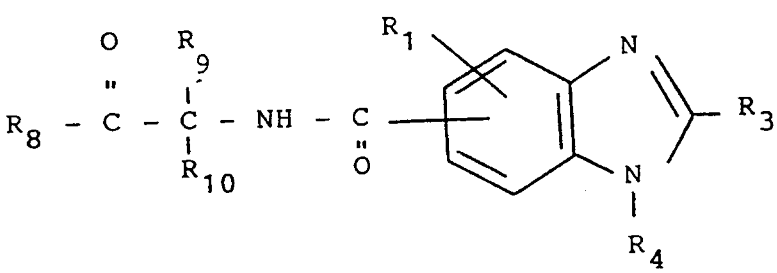

,(XVIII)

in der
$R_1$, $R_3$, $R_4$ und $R_8$ bis $R_{10}$ wie in den Ansprüchen 1 bis 5 definiert sind, wobei jedoch $R_9$ oder $R_{10}$ ein Wasserstoffatom darstellen muß, dehydratisiert wird oder

m) zur Herstellung von Verbindungen der allgemeinen Formel I, in der $R_2$ einen der durch die Reste $R_8$ bis $R_{10}$ substituierten in den Ansprüchen 1 bis 5 erwähnten Imidazol-2-yl-Reste darstellt, eine Verbindung der allgemeinen Formel

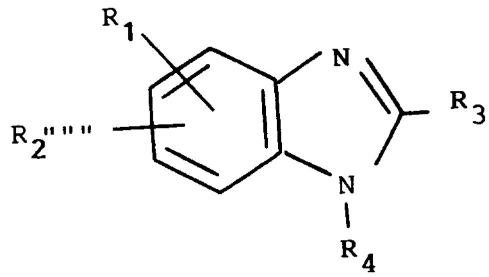

,(XIX)

in der
$R_1$, $R_3$ und $R_4$ wie in den Ansprüchen 1 bis 5 definiert sind und
$R_2$'''''' einen der durch die Reste $R_8$ bis $R_{10}$ substituierten in den Ansprüchen 1 bis 5 für $R_2$ erwähnten Imidazolin-2-yl-Reste darstellt, wobei jedoch $R_9$ oder $R_{10}$ ein Wasserstoffatom darstellen muß dehydriert wird oder
n) zur Herstellung von Verbindungen der allgemeinen Formel I, in der $R_5$ eine 2,5-Dihydro-5-oxo-1,2,4-oxadiazol-3-yl-Gruppe darstellt, ein gegebenenfalls im Reaktionsgemisch erhaltenes Amidoxim der allgemeinen Formel

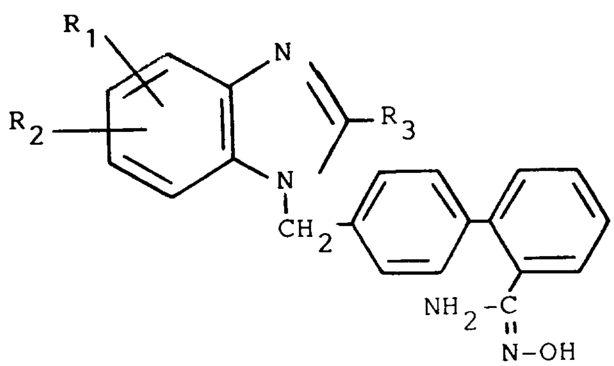

,(XX)

in der
$R_1$ bis $R_3$ wie in den Ansprüchen 1 bis 5 definiert sind, mit einer Verbindung der allgemeinen Formel

$Z_4$ - CO - $OR_{11}$ ,(XXI)

in der
$Z_3$ eine nukleophile Austrittsgruppe und
$R_{11}$ eine Alkyl-, Aryl- oder Aralkylgruppe bedeuten, umgesetzt und anschließend ein so erhaltenes acyliertes Amidoxim cyclisiert wird und
erforderlichenfalls ein während den Umsetzungen zum Schutze von reaktiven Gruppen verwendeter Schutzrest abgespalten wird oder
gewünschtenfalls anschließend eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER RECHERCHENBERICHT

Nummer der Anmeldung

EP 93 10 5770

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 400 835 (MERCK & CO, INC.) 5. Dezember 1990 *siehe Definitionen von R8a und R8b* | 1-10 | C07D403/04<br>C07D413/04<br>C07D413/14<br>C07D403/14<br>C07D235/08<br>A61K31/415<br>A61K31/42 |
| D,Y | EP-A-0 468 470 (DR. KARL THOMAE GMBH) 29. Januar 1992 *siehe Definitionen von R1* | 1-10 | |
| Y | EP-A-0 470 543 (DR. KARL THOMAE GMBH) 12. Februar 1992 *siehe Definitionen von R1* | 1-10 | |
| P,Y | EP-A-0 502 314 (DR. KARL THOMAE GMBH) 9. September 1992 *siehe Definitionen von R2* | 1-10 | |
| Y | EP-A-0 392 317 (DR. KARL THOMAE GMBH) 17. Oktober 1990 *siehe Definitionen von R1* | 1-10 | |

RECHERCHIERTE SACHGEBIETE (Int. Cl.5 )

C07D
A61K

Der vorliegende Recherchenbericht wurde für alle Patentansprüche erstellt

| Recherchenort | Abschlußdatum der Recherche | Prüfer |
|---|---|---|
| MUENCHEN | 16 JUNI 1993 | SCRUTON-EVANS I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)